# FASCICULE DE BREVET EUROPEEN

(11) **EP 1 981 846 B1**
(45) Date de publication et mention de la délivrance du brevet: **01.11.2017**
(21) Numéro de dépôt: 06794328.2
(22) Date de dépôt: 11.08.2006
(51) Int. Cl.: C07D 209/60

(54) **MARQUEURS, LEUR PROCEDE DE FABRICATION ET LEURS APPLICATIONS**
MARKER, VERFAHREN ZU IHRER HERSTELLUNG UND IHRE ANWENDUNG
MARKERS, METHOD FOR MANUFACTURING THEM AND THEIR APPLICATIONS

(30) Priorité: 11.08.2005 FR 0508525
(43) Date de publication de la demande: 22.10.2008
(73) Titulaire: Laboratoires Synth-Innove, F-75020 Paris (FR)
(72) Inventeur: SCHERNINSKI, François, F-75011 Paris (FR); GUYON, Vincent, F-78180 Montigny Le Bretonneux (FR); RAGER, Marie-Noëlle, F-75013 Paris (FR)
(74) Mandataire: Cabinet Plasseraud
(86) Numéro de dépôt international: PCT/FR2006/001947
(87) Numéro de publication internationale: WO 2007/017602

(56) Documents cités:
- EP-A- 0 502 723
- EP-A- 0 533 200
- EP-A- 0 940 681
- EP-A- 1 209 205
- EP-A- 1 491 590
- EP-A1- 0 800 831
- EP-A1- 1 273 584
- EP-A1- 1 493 781
- WO-A-00/63296
- WO-A-02/32466
- WO-A-90/02747
- WO-A-92/08720
- WO-A-95/07888
- WO-A-95/08772
- WO-A-96/10620
- WO-A-97/39064
- WO-A-98/30720
- WO-A-98/30992
- WO-A-99/07793
- WO-A-99/55805
- WO-A-03/082988
- WO-A-2004/011556
- WO-A-2004/039810
- WO-A-2004/065491
- WO-A-2005/019470
- WO-A-2005/058370
- WO-A1-00/16810
- FR-A- 2 748 027
- JP-A- 1 239 548
- JP-A- 3 228 046
- JP-A- 4 080 749
- US-A- 4 318 846
- US-A1- 4 424 201
- US-A1- 4 882 234
- US-A1- 5 627 027
- US-A1- 2002 077 487
- US-A1- 2005 037 332
- US-A1- 2005 158 804
- US-B1- 6 225 050
- US-B1- 6 291 162
- PATENT ABSTRACTS OF JAPAN -& JP 06 345794 A (TOA GOSEI KK), 20 décembre 1994 (1994-12-20)
- DATABASE BEILSTEIN Beilstein Institut zur Foerderung der chemischen Wissenschaften, Frankfurt am Main, DE; XP002415915 Database accession no. 8516712 & KOROTKIKH, N.I. ET AL.: CHEM. HETEROCYCL. COMPD., vol. 35, no. 3, 1999, pages 358-362,
- CATURLA, FRANCISCO ET AL: "New fluorescent probes for testing combinatorial catalysts with phosphodiesterase and esterase activities" TETRAHEDRON, vol. 60, no. 8, 2004, pages 1903-1911, XP002415885
- CAI, JIANFENG ET AL: "Nucleic acid-triggered fluorescent probe activation by the Staudinger reaction" JOURNAL OF THE AMERICAN CHEMICAL SOCIETY, vol. 126, no. 50, 2004, pages 16324-16325, XP002415886
- LOBNIK A ET AL: "pH optical sensors based on sol-gels. Chemical doping versus covalent immobilization" ANALYTICA CHIMICA ACTA, ELSEVIER, AMSTERDAM, NL, vol. 367, no. 1-3, 1998, pages 159-165, XP002382764 ISSN: 0003-2670 cité dans la demande
- KRAFFT, GRANT A. ET AL: "Photoactivable fluorophores. 3. Synthesis and photoactivation of fluorogenic difunctionalized fluoresceins" JOURNAL OF THE AMERICAN CHEMICAL SOCIETY, vol. 110, no. 1, 1988, pages 301-303, XP002415887
- DATABASE CA [Online] CHEMICAL ABSTRACTS SERVICE, COLUMBUS, OHIO, US; CORRIE, JOHN E. T. ET AL: "Synthesis of photoactivatable fluorescein derivatives bearing side chains with varying properties" XP002415916 extrait de STN Database accession no. 1995:772149 & JOURNAL OF THE CHEMICAL SOCIETY, PERKIN TRANSACTIONS 1: ORGANIC AND BIO-ORGANIC CHEMISTRY , (16), 1993-2000 CODEN: JCPRB4; ISSN: 0300-922X, 1995,
- DATABASE CA [Online] CHEMICAL ABSTRACTS SERVICE, COLUMBUS, OHIO, US; KASAI, KOUICHI ET AL: "Glycosides having chromophores as substrates for sensitive enzyme analysis. V. Synthesis of 6'-O-substituted 2',7'-dichlorofluorescein N-acetyl-.beta.-D-glucosaminides as substrates for the rate-assay of N-acetyl-.beta.-D-glucosaminidase" XP002415917 extrait de STN Database accession no. 1994:218340 & CHEMICAL & PHARMACEUTICAL BULLETIN , 41(9), 1513-20 CODEN: CPBTAL; ISSN: 0009-2363, 1993,
- MOREAU M-F ET AL: "SYNTHESE D'INDOMONOCARBOCYANINES A ELIMINATION BILIAIRE SELECTIVE ETUDE EXPERIMENTALE CHEZ L'ANIMAL" EUROPEAN JOURNAL OF MEDICINAL CHEMISTRY, EDITIONS SCIENTIFIQUE ELSEVIER, PARIS, FR, vol. 9, no. 3, mai 1974 (1974-05), pages 274-280, XP008070191 ISSN: 0223-5234
- DATABASE CA [Online] CHEMICAL ABSTRACTS SERVICE, COLUMBUS, OHIO, US; KOBAYASHI, SUGURU ET AL: "Silver halide photographic material containing sensitizing dye" XP002426743 extrait de STN Database accession no. 2003:628316 & JP 2003 228148 A (FUJI PHOTO FILM CO., LTD., JAPAN) 15 août 2003 (2003-08-15)
- DATABASE CA [Online] CHEMICAL ABSTRACTS SERVICE, COLUMBUS, OHIO, US; JACKSON, ANTHONY HUGH ET AL: "Pyrroles and related compounds. XIII. Porphyrin synthesis through b-oxobilanes and oxophlorins (oxyporphyrins)" XP002426744 extrait de STN Database accession no. 1968:59557 & JOURNAL OF THE CHEMICAL SOCIETY [SECTION] C: ORGANIC, vol. 3, 1968, pages 294-302,
- DATABASE CA [Online] CHEMICAL ABSTRACTS SERVICE, COLUMBUS, OHIO, US; KENNER, GEORGE W. ET AL: "Pyrroles and related compounds. XVI. Synthesis of protoporphyrin IX by the a- and b-oxobilane routes" XP002426745 extrait de STN Database accession no. 1971:112028 & JOURNAL OF THE CHEMICAL SOCIETY [SECTION] C: ORGANIC , (3), 487-502 CODEN: JSOOAX; ISSN: 0022-4952, 1971,
- DATABASE CA [Online] CHEMICAL ABSTRACTS SERVICE, COLUMBUS, OHIO, US; JACKSON, ANTHONY H. ET AL: "Synthesis of .gamma.-oxyprotoporphyrin IX and pterobiline (biliverdin IX.gamma.)" XP002426746 extrait de STN Database accession no. 1982:492246 & JOURNAL OF THE CHEMICAL SOCIETY, CHEMICAL COMMUNICATIONS, vol. 15, 1981, pages 763-764,
- DATABASE CA [Online] CHEMICAL ABSTRACTS SERVICE, COLUMBUS, OHIO, US; SMITH, KEVIN M. ET AL: "Novel porphyrins from copper(II)-mediated cyclizations of 1',8'-dimethyl-A,C-biladiene salts: mechanism of the cyclization reaction" XP002426747 extrait de STN Database accession no. 1985:470713 & JOURNAL OF ORGANIC CHEMISTRY , 50(12), 2073-80 CODEN: JOCEAH; ISSN: 0022-3263, 1985,
- DATABASE CA [Online] CHEMICAL ABSTRACTS SERVICE, COLUMBUS, OHIO, US; UENO, KEIHEI ET AL: "Preparation of metal-porphyrin complexes catalyzing oxidation reaction as labeling agents for trace detection of DNA and proteins" XP002426748 extrait de STN Database accession no. 1991:492309 & JP 03 038587 A (DOJINDO LABORATORIES, JAPAN) 19 février 1991 (1991-02-19)
- DATABASE CA [Online] CHEMICAL ABSTRACTS SERVICE, COLUMBUS, OHIO, US; PANDIAN, RAMASAMY P. ET AL: "Novel particulate spin probe for targeted determination of oxygen in cells and tissues" XP002426749 extrait de STN Database accession no. 2003:829066 & FREE RADICAL BIOLOGY & MEDICINE , 35(9), 1138-1148 CODEN: FRBMEH; ISSN: 0891-5849, 2003,
- DATABASE CA [Online] CHEMICAL ABSTRACTS SERVICE, COLUMBUS, OHIO, US; LU, FANLI ET AL: "Synthesis and characterization of novel sandwich-type lanthanide(III) complexes with mixed [tetrakis(4- chlorophenyl)porphyrinato] and [(.alpha.-octa- butoxy)phthalocyaninato] ligands" XP002426750 extrait de STN Database accession no. 2005:843665 & SYNTHESIS AND REACTIVITY IN INORGANIC, METAL-ORGANIC, AND NANO-METAL CHEMISTRY , 35(6), 463-467 CODEN: SRIMDO; ISSN: 1553-3174, 2005,

## Description

La présente invention a pour objet de nouveaux marqueurs utilisables pour le marquage de molécules cibles. L'invention concerne également un procédé pour l'obtention de ces marqueurs. Enfin, elle concerne l'application de ces nouveaux marqueurs pour la détection et/ou la quantification de molécules cibles.

Le marquage d'une molécule cible et notamment de molécules biologiques par des marqueurs présente un grand intérêt dans le domaine de la biologie cellulaire et moléculaire, en particulier en cytologie/histologie, cytométrie de flux, pour le séquençage d'acides nucléiques. Dans le domaine de l'imagerie médicale, l'utilisation de marqueurs fluorescents permet entre autre la localisation de lésions ou de tumeurs.

Les marqueurs fluorescents sont largement utilisés en biologie, biophysique, ou physiologie non seulement pour étudier les processus biologiques au niveau cellulaire et moléculaire ou quantifier des substances physiologiques à analyser mais également en médecine dans des techniques d'imagerie non invasives pour la surveillance ou le diagnostic.

Quelques exemples spécifiques d'application de marqueurs fluorescents en biologie sont :
- l'identification et la séparation de sous population de cellules dans des mélanges de cellules par les techniques de cytométrie de flux ou de microscopie,
- la détermination de la concentration d'une substance qui se fixe sur une seconde espèce telle qu'un anticorps sur un antigène dans des techniques d'essai immunologique, le séquençage des acides nucléiques, la mise en évidence de l'appariement de séquences de nucléotides,
- la localisation de substances telles que l'ADN, les protéines dans des gels d'électrophorèse ou d'autres supports insolubles par des techniques de coloration fluorescente.

Dans de nombreuses techniques, dont la microscopie optique en fluorescence, il est possible d'utiliser simultanément plusieurs marqueurs fluorescents capables d'émettre à des longueurs d'ondes différentes. Ce "multi-marquage" est réalisé sans qu'aucune confusion entre les signaux ne se produise lors de la détection.

Le principe du multi-marquage est également utilisé dans la technique particulière du FRET *(Fluorescence Resonance Energy Transfer)* qui exploite le transfert d'énergie de l'état excité d'un marqueur fluorescent donneur vers un deuxième marqueur fluorescent accepteur, ce transfert d'énergie n'étant possible que lorsque les deux marqueurs sont à proximité. Cette technique permet de mesurer les interactions (association ou dissociation) entre deux protéines, dont l'une est couplée à un marqueur fluorescent donneur et l'autre est couplée à un marqueur fluorescent accepteur.

Toutefois, lors de ces multi-marquages, il faut tenir compte des problèmes de chevauchements partiels éventuels des spectres d'émission des marqueurs utilisés qui faussent la lecture des résultats. Par conséquent, lors du multi-marquage, il est primordial de limiter le phénomène de recouvrement des spectres, ce qui impose d'avoir accès à une large gamme de marqueurs dont les caractéristiques spectrales sont totalement maîtrisées.

Le premier objet de la présente invention consiste en de nouveaux marqueurs composés de "colorants fonctionnalisés" dont le système de résonance n'est pas affecté par la fonctionalisation.

Par "colorant" on entend toute substance colorée, naturelle, artificielle ou synthétique, absorbant la lumière dans le domaine de l'ultra-violet, le visible et/ou l'infrarouge. Dans le cadre de l'invention, les colorants peuvent être fluorescents. Par "colorants fluorescents", on désigne des colorants ayant la faculté d'être excités de façon transitoire par absorption d'une radiation lumineuse puis de revenir à leur état initial en émettant une radiation dont la longueur d'onde est plus élevée que celle de la radiation d'excitation.

Par "colorants fonctionnalisés", on désigne des colorants possédant au moins une fonction chimique réactive permettant leur couplage à des molécules cibles et éventuellement au moins une fonction leur permettant d'être solubles dans des conditions d'utilisation.

Les marqueurs de l'art antérieur peuvent être classés en trois catégories selon la façon dont leur fonction chimique réactive est liée à la structure cyclique du colorant.

Les marqueurs de la première catégorie se définissent par le fait que leur fonction chimique réactive est reliée directement à un atome de carbone d'un cycle du squelette hydrocarboné du colorant. Ils ont été décrits notamment dans US5,627,027, US2002/077487, FR2764605, US4,614,723, JP03133629, JP03133628, JP63277680, Masiero, S. et al. « G-quartets as a self-assembled scaffold dor circular porphyrin arrays » Chemical communications, vol.15, 2003 pages 1995-1996, Yu, Junhua et al. « Porphyrin capped TiO2 nanoclusters, tyrosine methyl ester enhanced electron transfer », FR2 748 027.

Cependant, l'inconvénient majeur de tels marqueurs vient du fait que la fonction chimique réactive se trouve à proximité immédiate d'un cycle de la molécule du colorant. Cette proximité entraîne un encombrement stérique important qui gêne fortement le couplage à des molécules cibles. D'autre part cette proximité affecte les spectres d'absorption et de fluorescence du colorant. Or le choix d'un colorant pour la synthèse d'un marqueur est dicté par ses propriétés spectrales. Par conséquent, l'obtention d'un marqueur dont la faculté de couplage est gênée par un encombrement stérique et dont les propriétés spectrales sont différentes de celles du colorant choisi n'est pas particulièrement intéressante.

Afin de remédier aux inconvénients précédemment cités, une deuxième catégorie de marqueurs a été développée.

Les marqueurs de cette deuxième catégorie se définissent par le fait que leur fonction chimique réactive est reliée indirectement par l'intermédiaire d'une chaîne alkyle à un atome qui appartient à la molécule du colorant initial, cet atome n'étant pas un atome de carbone. Il s'agit fréquemment d'un atome d'azote qui, le plus souvent, appartient à la structure cyclique de ce colorant.

La fabrication de ces marqueurs de deuxième catégorie est limitée à l'utilisation de colorants constitués le plus souvent d'un ou plusieurs hétérocycles azotés. Chaque atome d'azote du colorant initial porte une chaîne qui influence fortement la solubilité du colorant : il s'agit souvent de chaînes sulfoalkyle si le colorant doit être solubilisé en milieu aqueux ou de chaînes alkyle si le colorant doit être solubilisé en milieu hydrophobe. La technique de réalisation des marqueurs de cette deuxième catégorie consiste à remplacer une ou plusieurs de ces chaînes alkyle ou sulfoalkyle par une chaîne hydrocarbonée portant la fonction chimique réactive. Néanmoins, cette technique a pour conséquence de réduire notablement la solubilité du marqueur relativement aux caractéristiques initiales du colorant. De plus, la fabrication des marqueurs de cette deuxième catégorie ne donne accès qu'à un nombre très limité d'entités car elle n'est applicable qu'à un nombre restreint de colorants qui comportent un ou plusieurs hétéroatomes.

Par exemple, les brevets WO2005/058370, WO2003/082988, EP1209205 US 6,027,709 et US 6,224,644 divulguent des marqueurs de cette deuxième catégorie.

Le brevet US 6,027,709 décrit des colorants de type carbocyanine dans lesquels une chaîne sulfoalkyle a été remplacée par une chaîne alkyle portant une fonction acide carboxylique (fonction chimique réactive à caractère électrophile).

Le brevet US 6,224,644 décrit également des carbocyanines modifiées de façon similaire et destinées à être utilisées comme marqueurs de molécules biologiques. Toutefois, ces marqueurs se distinguent de ceux du brevet US 6,027,709 par le fait que la fonction chimique réactive n'est pas électrophile mais nucléophile (fonction hydroxyle, amine et thiol).

Cette catégorie de marqueurs présente donc un intérêt limité car elle ne s'applique qu'à quelques colorants de type carbocyanine avec une réduction de solubilité du marqueur relativement à celle du colorant initial alors qu'il aurait été souhaitable d'augmenter cette solubilité pour faciliter le couplage avec un grand nombre de molécules cibles. Dans le cas où l'hydrosolubilité du marqueur est souhaitée, l'adjonction directe de groupements tels que NO₂ et SO₃⁻ sur les cycles aromatiques remédie à ce problème mais affecte les spectres d'absorption et de fluorescence du colorant initial.

Une troisième catégorie de marqueurs, tels que décrits dans EP 1 209 205, WO02/32466, WO94/08631 et Lobnik, A. et al. « pH optical sensors based on sol-gels. Chemical doping versus covalent immobilization » Analytica Chimica Acta, vol.367, no.1-3, 1998, pages 159-165, est définie par le fait que leur fonction chimique réactive est reliée indirectement par l'intermédiaire d'une chaîne alkyle ou amidoalkyle à un atome de carbone, et non pas un atome d'azote, de la structure cyclique du colorant, ce qui évite la suppression d'une des chaînes sulfoalkyle ou alkyle qui conditionnent la solubilité du marqueur.

Néanmoins, la fabrication des marqueurs de cette troisième catégorie donne accès à un nombre d'entités encore plus limité que ceux de la deuxième catégorie car ils ont l'inconvénient majeur d'être difficilement synthétisables. En effet, les précurseurs de synthèse de tels marqueurs ne sont pas couramment disponibles dans le commerce et leur synthèse nécessite la mise en oeuvre de nombreuses étapes avec de faibles rendements finaux.

A ce jour, la synthèse de colorants fonctionnalisés servant de marqueurs pour détecter et ou quantifier des molécules cibles est connue. Toutefois, les marqueurs de l'art antérieur ont au moins l'un des trois inconvénients fondamentaux suivants, qui limitent considérablement leur utilisation :
- la fonction chimique réactive de ces marqueurs est à proximité immédiate d'un cycle du colorant, ce qui entraîne un encombrement stérique et gêne fortement le couplage à des molécules cibles, les rendant ainsi peu utilisables. Par ailleurs, leurs propriétés spectrales sont différentes de celles des colorants à partir desquels ils sont obtenus, en raison des effets électroniques qui se produisent entre la fonction chimique réactive et les cycles aromatiques du colorant.
- Ces marqueurs sont difficilement solubles dans le milieu de couplage dont la nature est dictée par l'identité de la molécule cible.
- Ces marqueurs ne sont pas synthétisables à partir de réactifs facilement accessibles dans le commerce et selon des procédés de synthèse simples et rapides ; de ce fait le nombre disponible de ces marqueurs est limité.

La présente invention se propose quant à elle de préparer selon un procédé particulier des marqueurs d'une quatrième catégorie ne présentant aucun des inconvénients des marqueurs de l'art antérieur.

Par ailleurs, à la connaissance de la demanderesse, il n'a jamais été décrit, dans l'art antérieur, un procédé permettant de fonctionnaliser aisément un très large panel de colorants. En effet, les procédés de l'art antérieur ne peuvent s'appliquer qu'à un nombre restreint de colorants alors qu'il existe un grand nombre de colorants ayant des caractéristiques spectrales spécifiques présentant un intérêt pour le marquage de molécules cibles.

De plus, lors des multi-marquages, il est essentiel de tenir compte des éventuels problèmes de chevauchements partiels des spectres d'émission des marqueurs utilisés qui faussent la lecture des résultats. Dans le cadre de la présente invention, ce problème de recouvrement est minimisé dans la mesure où l'utilisateur dispose d'un large panel de marqueurs, éventuellement fluorescents ayant des propriétés spectrales différentes, c'est-à-dire des longueurs d'onde d'excitation et d'émission différentes couvrant une large région du spectre de détection.

L'intérêt du procédé de l'invention est de permettre de sélectionner un colorant en fonction de ses caractéristiques spectrales particulières et de pouvoir aisément le fonctionnaliser sans altérer notablement ses caractéristiques spectrales ni ses caractéristiques de solubilité.

Au regard de l'art antérieur, il est apparent qu'il existe un besoin pour une grande variété de marqueurs faciles à synthétiser à partir de colorants dont le choix est principalement dicté par leurs propriétés d'absorption et d'émission, lesdits marqueurs ayant non seulement sensiblement les mêmes propriétés spectrales que les colorants à partir desquels ils sont obtenus mais également une solubilité dans le milieu de couplage qui soit équivalente voire améliorée relativement au colorant initial.

Les inventeurs, à l'issue de recherches approfondies, ont eu le mérite de trouver qu'il était possible d'obtenir un large panel de nouveaux marqueurs regroupant toutes les caractéristiques fonctionnelles et spectrales recherchées à partir d'un large panel de colorants ou d'intermédiaires couramment disponibles commercialement.

La présente invention est telle que décrite dans le jeu de revendications annexé.
Il est aussi décrit de nouveaux marqueurs susceptibles de former une liaison covalente ou non covalente avec une molécule cible, consistant en un colorant auquel est lié de façon covalente par un ou plusieurs carbones de sa structure chimique :
un ou plusieurs groupe(s) [FONC], et
éventuellement un ou plusieurs groupe(s) [SOL],
ledit marqueur présentant la formule générale : 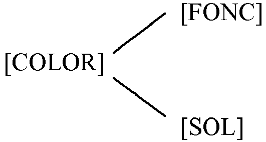
[COLOR] représentant une molécule choisie dans le groupe comprenant les phtaléines, les carbocyanines, les mérocyanines, les porphyrines, les phtalocyanines ;
[FONC] représentant chacun indépendamment un groupe -X-A-Z, dans lequel :
   - X est choisi dans le groupe consistant en un atome d'oxygène, un atome de soufre, un groupe NR₁R₂, R₁ et R₂ étant chacun indépendamment l'un de l'autre un atome d'hydrogène ou un groupe alkyle, linéaire ou ramifié en C₁-C₃₀, de préférence en C₁-C₁₈ et plus préférentiellement en C₁-C₅ ;
      A est choisi dans le groupe consistant en un groupe alkylène ou un groupe alkylène-arylène ;
   - Z est une fonction chimique réactive ;
[SOL] représentant chacun indépendamment un groupe -X'-A'-Z', dans lequel :
   - X' est choisi dans le groupe consistant en un atome d'oxygène, un atome de soufre, un groupe NR₁R₂, R₁ et R₂ étant chacun indépendamment l'un de l'autre un atome d'hydrogène ou un groupe alkyle, linéaire ou ramifié en C₁-C₃₀, de préférence en C₁-C₁₈ et plus préférentiellement en C₁-C₅ ;
      A' est choisi dans le groupe consistant en un groupe alkylène, ou un groupe alkylène-arylène;
   - Z' est un groupement polaire ou apolaire.

Par "phtaléines" on entend les colorants présentant la structure (1) suivante : 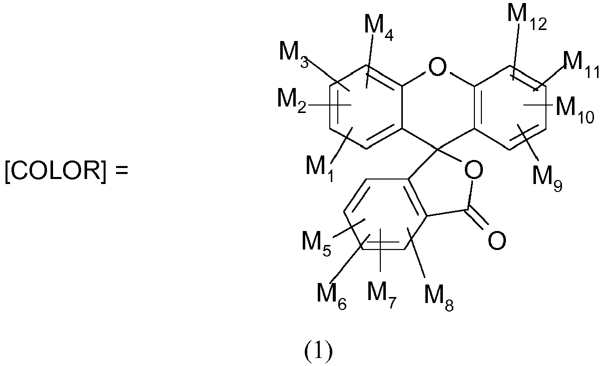 par "carbocyanines" on entend les colorants présentant la structure (2) suivante : 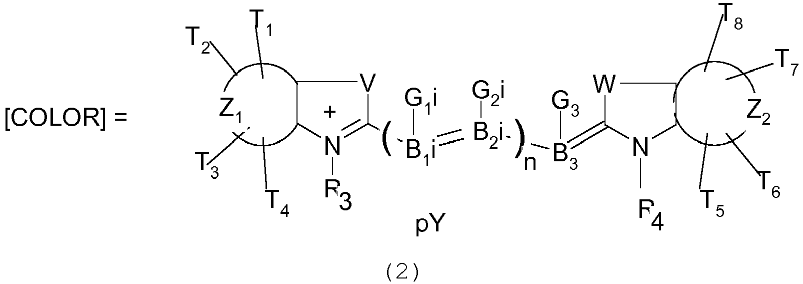 par "mérocyanines" on entend les colorants présentant la structure (3) suivante : 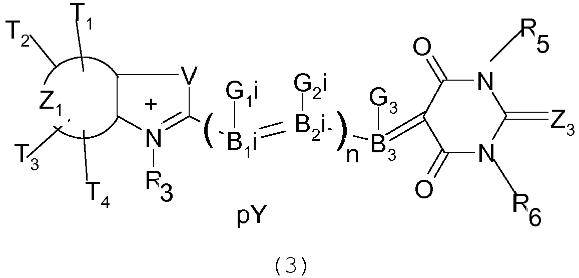 par "porphyrines" on entend les colorants présentant la structure (4) suivante : 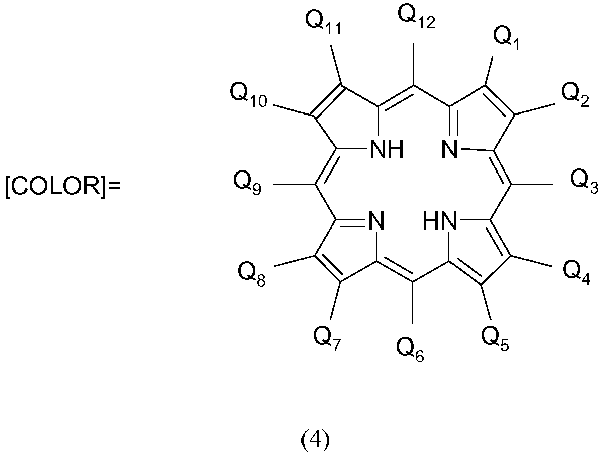 par « phtalocyanines » on entend les colorants présentant la structure (5) suivante : 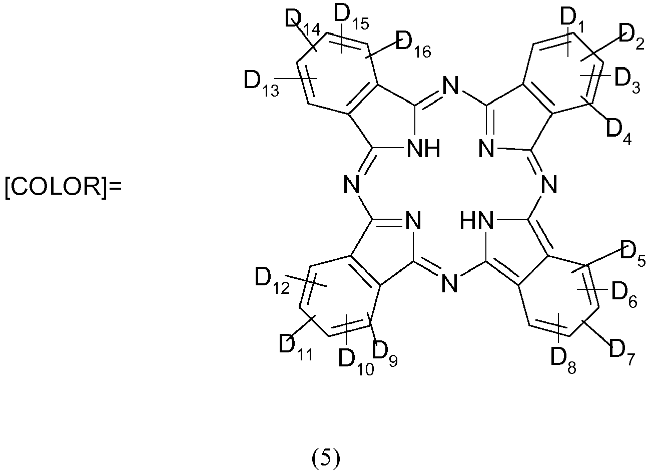 dans lesquelles :
- M₁ à M₄, M₉ à M₁₂, Q₁ à Q₁₂, D₁ à D₁₆, qui sont identiques ou différents les uns des autres, sont choisis dans le groupe comprenant les radicaux ou groupements suivants : hydrogène, hydroxyle, halogène, acétyle, amine, amine substituée, ammonium quaternaire, phosphate, nitro, acide carboxylique et ses sels, acide sulfonique et ses sels, alkylcarboxy de 2 à 30 atomes de carbone, alkyle, linéaire ou ramifié, ayant de 1 à 30 atomes de carbone, cycloalkyle ayant de 3 à 14 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, nitroalkyle ayant de 1 à 30 atomes de carbone, carboxyalkyle ayant de 2 à 30 atomes de carbone, aminoalkyle ayant de 1 à 30 atomes de carbone, sulfoalkyle ayant de 1 à 30 atomes de carbone, aryle, aryloxy, arylalkyle, halogénoaryle, arylester,
   Q₃, Q₆, Q₉ et Q₁₂ ne pouvant pas représenter aryle ou aryloxy,
   à la condition que l'un au moins parmi Q₁ à Q₁₂ représente H ou un cycle aromatique,
   à la condition que l'un au moins parmi D₁ à D₁₆ représente H ;
- Z₁ et Z₂ représentent chacun indépendamment l'un de l'autre les atomes nécessaires pour compléter un noyau indole, benzindole ou naphtindole ;
- Z₃ représente O ou S ;
   - V et W sont chacun indépendamment l'un de l'autre choisis parmi CR₇R₈, O, S, Se et NR₉, où R₇, R₈ et R₉ sont chacun indépendamment les uns des autres choisis parmi l'hydrogène et un groupe (CH₂)ₘR₁₀, ou m est un nombre entier de 1 à 18 et R₁₀ est sélectionné parmi hydrogène, amine, amine substituée, ammonium quaternaire, aldéhyde, halogène, cyano, aryl, heteroaryl, hydroxyl, amide, acide sulfonique et ses sels, acide carboxylique et ses sels ;
   - n est un nombre entier de 1 à 10, de préférence de 1 à 7 et plus préférentiellement de 1 à 3 ;
   - i est un nombre entier de 1 à n;
   - R₃ à R₆ représentent chacun indépendamment les uns des autres un atome d'hydrogène, un groupe alkyle, linéaire ou ramifié en C₁-C₃₀, de préférence en C₁-C₁₈, plus préférentiellement en C₁-C₅, cycloalkyle, aryle, aryloxy, nitroalkyle, alkylamine, alkylamine substituée, alkylammonium quaternaire, alkylphosphate, acide alkylsulfonique et ses sels ;
   - T₁ à T₈, représentent chacun indépendamment les uns des autres un atome d'hydrogène, un atome d'halogène, un groupe alkyle linéaire ou ramifié en C₁-C₃₀, de préférence en C₁-C₁₈, plus préférentiellement en C₁-C₅, sulfoalkyle, cycloalkyle, aryle, aryloxy, nitro, amine, amine substituée, ammonium quaternaire, phosphate, acide sulfonique et ses sels, OR₁₁ avec R₁₁ choisi parmi l'hydrogène et un groupe alkyle en C₁-C₃₀, de préférence en C₁-C₁₈, plus préférentiellement en C₁-C₅, COOR₁₁ ou CONHR₁₁ avec R₁₁ tel que défini précédemment ;
   - G₁ᵢ, G₂ᵢ et G₃ et M₅ à M₈ représentent chacun indépendamment les uns des autres un atome d'hydrogène, un groupe alkyle linéaire ou ramifié en C₁-C₃₀, de préférence en C₁-C₁₈, plus préférentiellement en C₁-C₅, cycloalkyle, aryle ;
   - B₁ᵢ, B₂ᵢ, B₃ représentent chacun indépendamment les uns des autres un groupement méthine (=CH-), cycloalkyle mono ou di-insaturé de 4 à 8 atomes de carbone, aryle-cycloalkyle mono ou di-insaturé de 4 à 8 atomes de carbone, ou un des groupements suivants : 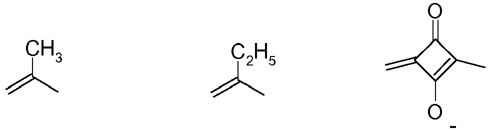 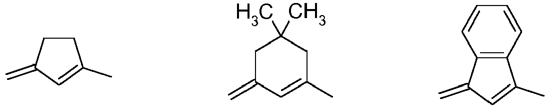 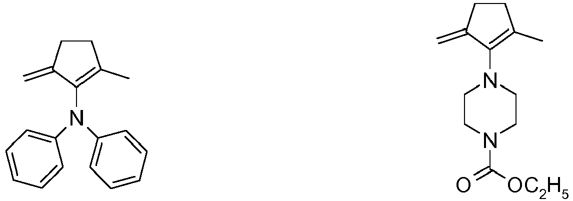
   chacun de ces groupes étant soit non substitué, soit substitué par un ou plusieurs des groupes suivants : alkyle linéaire ou ramifié en C₁-C₁₈, de préférence C₁-C₅, halogène, sulfonate, sulfoalkyle, aryle, sulfoaryle, aryloxy, hydroxy, hydroxylate, cétone, nitro, amine, amine substituée, ammonium quaternaire ;
- Y représente un contre-ion choisi parmi les ions suivants : halogénure, p-toluènesulfonate, méthanesulfonate, trifluorométhane-sulfonate, perchlorate, acétate, sodium, potassium, calcium, magnésium, lithium, ammonium et trialkylammonium ;
- p est un nombre entier de 0 à 8 nécessaire à la neutralité de la molécule.

Par l'expression "fonction chimique réactive", on désigne tout groupe fonctionnel capable de se lier par liaison covalente ou non covalente (électrostatique, hydrogène, coordinative, ionique ou complexe) directement ou après activation, avec au moins l'une des fonctions naturellement présentes ou artificiellement introduites sur une molécule cible. A titre d'exemples non limitatifs de fonctions chimiques réactives appropriées aux fins de l'invention, on peut citer notamment les fonctions acide carboxylique et ses sels, acide sulfonique et ses sels, anhydride d'acide, chlorure d'acide, ester (alkyl ester, p-nitrophényl ester, succinimidyl ester, sulfosuccinimidyl ester, etc...), azido (azoture d'acyle, azidonitrophényl, etc...), hydrazide, 3-acyl-1,3-thiazolidine-2-thione, amine, amine substituée, ammonium quaternaire, isocyanate, isothiocyanate, hydrazine, phtalimido, maléimide, haloacétamide, monochlorotriazine, dichlorotriazine, pyridine mono ou dihalogénée, diazine mono ou dihalogénée, aziridine, thiol, chlorure de sulfonyle, vinylsulfone, disulfure, méthanethiosulfonate, hydroxyle, phosphoramidite, époxy, aldéhyde, carbonate, glyoxal, imidazolyle.

Par l'expression "groupement polaire ou apolaire", on désigne tout groupe fonctionnel capable de faciliter la solubilisation d'un composé chimique dans les solvants polaires ou apolaires. A titre d'exemples non limitatifs de groupements polaires, on peut citer notamment les groupes, acide carboxylique et ses sels, acide sulfonique et ses sels, amine, amine substituée, ammonium quaternaire, carbohydrate, glycol, hydroxyle, nitro, phosphate. A titre d'exemples non limitatifs de groupements apolaires on peut citer notamment les groupes alkyle linéaire ou ramifié ayant de 1 à 30 atomes de carbone, aryle, cycloalkyle ayant de 3 à 14 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, aryloxy, arylalkyle, arylalkyle substitué, halogénoaryle.

Par « alkylène », on désigne une chaîne hydrocarbonée, cyclique, linéaire ou ramifiée, présentant deux liaisons libres, contenant de 1 à 30 atomes de carbone, de préférence de 1 à 18 atomes de carbone et plus préférentiellement encore de 1 à 5 atomes de carbone et pouvant être notamment une chaîne méthylène, éthylène, propylène, 2-méthylpropylène, n-butylène, i-pentylène, n-pentylène, hexylène, heptylène, octylène, nonylène, ou un groupe cyclopentadiène.

Par "arylène", on désigne un groupement aromatique, présentant deux liaisons libres, contenant un ou plusieurs cycles aromatiques, éventuellement substitué dont notamment un phénylène pouvant être substitué ou non substitué.

Par « alkyle », et « alkyle en C₁-C₃₀ » on désigne une chaîne hydrocarbonée, cyclique, linéaire ou ramifiée, présentant une liaison libre, contenant de 1 à 30 atomes de carbone, de préférence de 1 à 18 atomes de carbone et plus préférentiellement encore de 1 à 5 atomes de carbone et pouvant être notamment une chaîne méthyl, éthyl, n-propyl, iso-propyl, n-butyl, iso-butyl, sec-butyl, tert-butyl, n-pentyl, 1-méthylbutyl, 2,2-diméthylbutyl, 2-méthylpentyl, 2,2-diméthylpropyl, isopentyl, néopentyl, 2-pentyl, hexyl, 2-hexyl, 3-hexyl, 3-méthylpentyl, heptyl, octyl, nonyl, décyl, dodécyl.

Par "molécule cible", on désigne une molécule biologique ou non biologique destinée à être couplée à un marqueur. Cette expression inclut mais n'est pas limitée à des molécules organiques, des polymères, des matériaux silicates, naturels ou synthétiques, des vésicules lipidiques, des acides aminés, des acides nucléiques, des nucléotides, des oligonucléotides, des peptides, des protéines, des carbohydrates, des oligosaccharides, des polysaccharides, des anticorps, des antigènes, des récepteurs cellulaires, des haptènes, des pectines, des cytokines, des hormones, des toxines, des bactéries, des virus, des cellules eucaryotes.

Le marqueur conforme à l'invention est caractérisé par le fait que chacun des groupes [FONC] ou des groupes [SOL] est fixé à un carbone de la structure chimique du colorant par l'intermédiaire de son groupe respectif X ou X' tels que définis dans la revendication 1. De ce fait, les groupements hydrophiles ou hydrophobes initialement présents sur le colorant et qui peuvent être portés par les atomes d'azote ou par tout autre atome de la structure, ne sont pas modifiés.

Des exemples particuliers mais non limitatifs de groupe [FONC] sont donnés ci-après (Su représente le groupement succinimidyl) :
-X-(CH₂)ᵣ-COOSu, -X-(CH₂)ᵣ-COOSuSO₃Na, -X-(CH₂)ᵣ-COOH, -X-(CH₂)ᵣ-SO₃H, -X-(CH₂)ᵣ-SO₃Na, -X-(CH₂)-COO-C₆H₄-NO₂, -X- (C₆H₄)-(CH₂)ᵣ-COOSu, -X- (CH₂)ᵣ-NHCOCH₂I, -X- (CH₂)ᵣ-NCS, -X- (CH₂)ᵣ-C₆H₄CH(CH₃)-COOSu, -N(CH₃)₂-(CH₂)ᵣ-SO₃H,
-X-(CH₂)ᵣ-OP[N(iPr)₂][CH₂CH₂CN], X est tel que défini précédemment, r est un nombre entier de 1 à 18, de préférence de 2 à 10, et plus préférentiellement de 3 à 5.

De façon générale, les conditions de réaction de couplage entre un marqueur et une molécule cible sont dictées par la nature de la molécule cible. Dans le cas du marquage de certaines molécules biologiques fragiles, notamment de protéines, il est préférable que la réaction de couplage se fasse en solution aqueuse pour éviter la dénaturation de celles-ci. A cet effet, il est intéressant que le marqueur soit doté d'un ou plusieurs groupes Z' lui conférant un caractère hydrosoluble dans le milieu de couplage non dénaturant pour la protéine. Par contre, pour d'autres molécules cibles des groupes hydrophobes ou non polaires sont préférables. Dans ce cas, le marqueur doit posséder préférentiellement au moins un groupe Z' lui conférant un caractère hydrophobe ou non polaire.

Ces groupes hydrophiles ou lipophiles (Z') peuvent éventuellement être introduits de la même façon que la fonction chimique réactive Z. Des exemples particuliers mais non limitatifs de groupe [SOL] comportant un groupe Z', hydrophile ou lipophile, illustrant cette analogie structurelle sont donnés ci-après:
- X-(CH₂)ᵣ-SO₃Na, -X-(CH₂)ᵣ-SO₃H, -X-(CH₂)ᵣ-C₆H₃(NO₂)₂, -X-(CH₂)ᵣ-CH₃, X est tel que défini précédemment, r est un nombre entier de 1 à 18, de préférence de 2 à 10, et plus préférentiellement de 3 à 5.

Les marqueurs de l'invention peuvent être fluorescents, lorsqu'ils sont réalisés à partir de colorants fluorescents, et présenter des caractéristiques spectrales fluorescentes pouvant aller de l'ultraviolet à l'infrarouge.

Ainsi, il est décrit des marqueurs dérivant de colorants choisis dans le groupe comprenant les colorants suivants : les phtaléines, les carbocyanines, les mérocyanines, les porphyrines, les phtalocyanines lesdits colorants étant tels que définis précédemment.

Un marqueur obtenu à partir d'une phtaléine, est un composé présentant la structure (6), 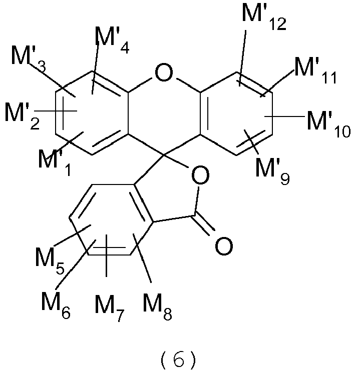 dans laquelle :
chacun de M₅ à M₈ est tel que défini précédemment ;
chacun de M'₁ à M'₄ et M'₉ à M'₁₂, indépendamment l'un de l'autre, peut avoir la définition donnée précédemment pour M₁ à M₄ et M₉ à M₁₂, ou peut représenter [SOL] ou [FONC], [SOL] et [FONC] étant tels que définis précédemment, et à la condition que l'un au moins parmi M'₁ à M'₄ et M'₉ à M'₁₂ représente [FONC].

Selon un mode de réalisation particulier du marqueur de structure (6), l'un au moins parmi M'₁, M'₂, M'₃ ou M'₄ représente [FONC], et/ou l'un au moins parmi M'₉, M'₁₀, M'₁₁ ou M'₁₂ représente [FONC].

Le marqueur conforme à l'invention de type carbocyanine est tel que décrit dans la revendication 1.
Il est aussi décrit un composé présentant la structure (7) suivante 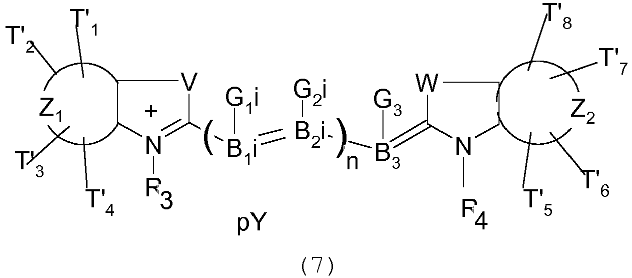 dans laquelle
Z₁, Z₂, R₃, R₄, B₁ᵢ, B₂ᵢ, B₃, G₁ᵢ, G₂ᵢ, G₃ ,V, W, Y, i, n, et p sont tels que définis précédemment,
chacun de T'₁ à T'₈, indépendamment l'un de l'autre, peut avoir la définition donnée précédemment pour T₁ à T₈, ou peut représenter [SOL] ou [FONC], [SOL] et [FONC] étant tels que définis précédemment, et à la condition que l'un au moins parmi T'₁ à T'₈ représente [FONC].

Selon un mode de réalisation particulier du marqueur de structure (7), au moins l'un parmi T'₁ à T'₄ et/ou au moins l'un parmi T'₅ à T'₈ représente [FONC].

Le marqueur de type merocyanine est un composé présentant la structure (8) suivante : 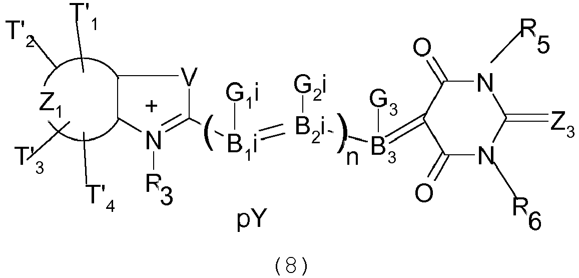 dans laquelle
Z₁, Z₃, R₃, R₅, R₆, G₁ᵢ, G₂ᵢ et G₃, B₁ᵢ, B₂ᵢ, B₃, V, Y, i, n et p sont tels que définis précédemment,
chacun de T'₁ à T'₄, indépendamment l'un de l'autre, peut avoir la définition donnée précédemment pour T₁ à T₄, ou peut représenter [SOL] ou [FONC], [SOL] et [FONC] étant tels que définis précédemment, et à la condition que l'un au moins parmi T'₁ à T'₄, représente [FONC].

Le marqueur de type porphyrine est un composé présentant la structure (9) suivante : 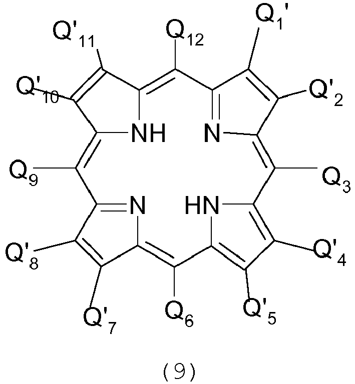 dans laquelle
Q₃, Q₆, Q₉ et Q₁₂ sont tels que définis précédemment,
chacun de Q'₁, Q'₂, Q'₄, Q'₅, Q'₇, Q'₈, Q'₁₀ et Q'₁₁ indépendamment l'un de l'autre peut avoir la définition donnée précédemment pour Q₁, Q₂, Q₄, Q₅, Q₇, Q₈, Q₁₀ et Q₁₁ ou peut représenter [SOL] ou [FONC], [SOL] et [FONC] étant tels que définis précédemment, et à la condition que l'un au moins de Q'₁, Q'₂, Q'₄, Q'₅, Q'₇, Q'₈, Q'₁₀ et Q'₁₁ représente [FONC].

Le marqueur de type phtalocyanine est un composé présentant la structure (10) suivante : 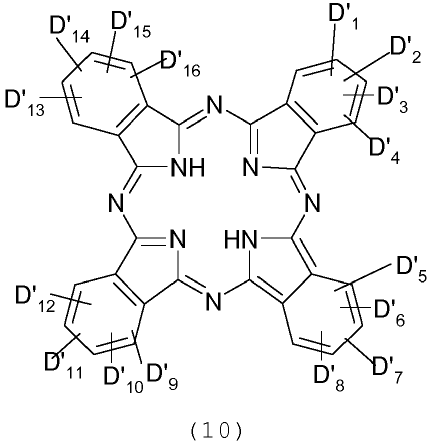 dans laquelle
chacun de D₁' à D₁₆' , indépendamment l'un de l'autre, peut avoir la définition donnée précédemment pour D₁ à D₁₆, ou peut représenter [FONC] ou [SOL], [FONC] et [SOL] étant tels que définis précédemment, et à la condition que l'un au moins parmi D'₁ à D'₁₆ représente [FONC].

Selon un mode de réalisation de l'invention, le marqueur est hydrosoluble, de préférence à une concentration supérieure à 0,5% (m/v), plus préférentiellement à une concentration supérieure ou égale à 2% (m/v), et encore plus préférentiellement encore supérieure à 10% (m/v).

Selon un autre mode de réalisation de l'invention, le marqueur est liposoluble, de préférence à une concentration supérieure à 0,5% (m/v), plus préférentiellement à une concentration supérieure ou égale à 2% (m/v), et encore plus préférentiellement encore supérieure à 10% (m/v).

Des exemples spécifiques de marqueurs précédemment décrits sont les composés suivants :
(1) 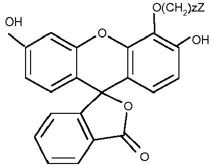 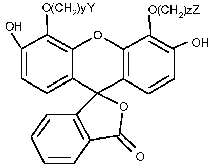
   dans laquelle chacun de y, z identiques ou différents, est un entier égal à 1, 2, 3, 4, 5, 6, 7 ou 8,
   Z représente-COOH ou 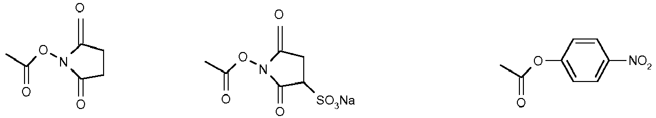
   Y représente SO₃⁻ ou SO₃Na
(2) 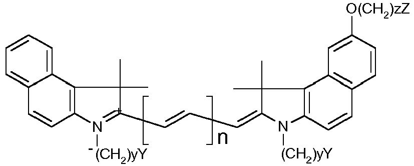
   dans laquelle chacun de y, z et n, identiques ou différents, est un entier égal à 1, 2, 3, 4, 5, 6, 7 ou 8, Z représente-COOH ou 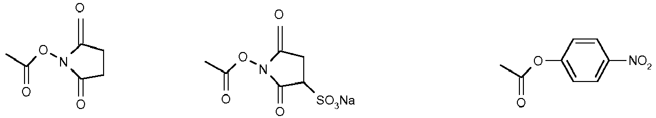
   Y représente SO₃⁻ ou SO₃Na
      en particulier 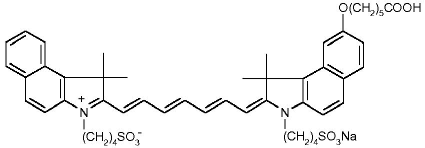 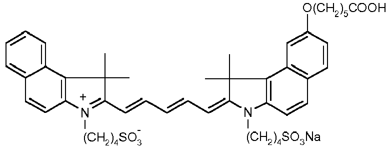 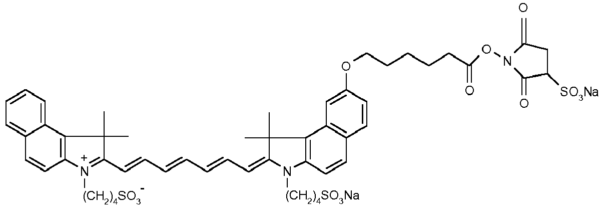 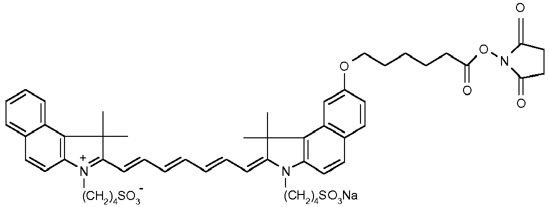 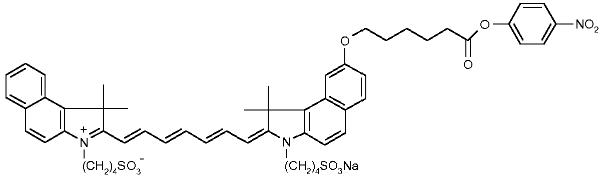 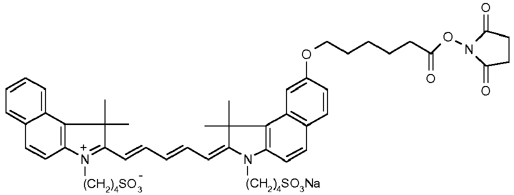 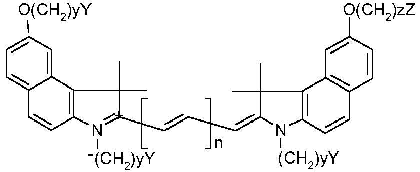
   dans laquelle chacun de y, z et n, identiques ou différents, est un entier égal à 1, 2, 3, 4, 5, 6, 7 ou 8
   Z représente-COOH ou 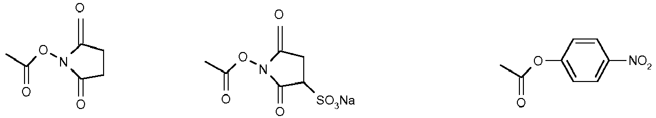
   Y représente SO₃⁻ ou SO₃Na
      en particulier 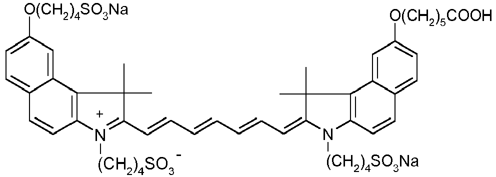 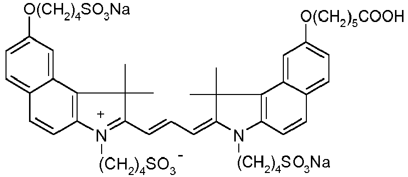 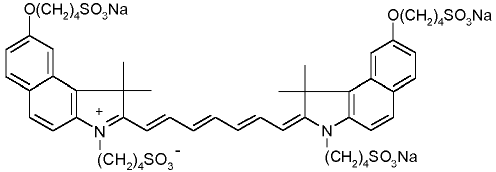 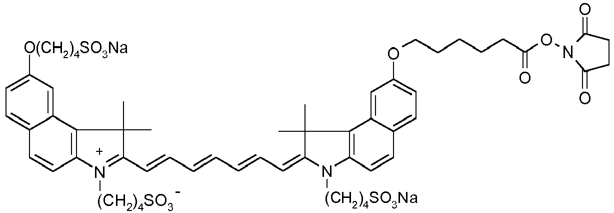
(3) 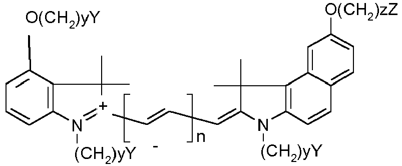 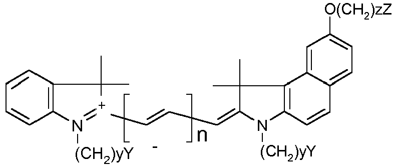 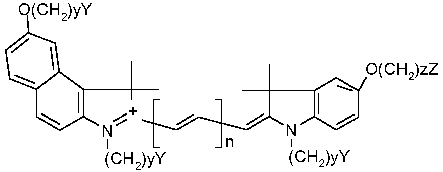 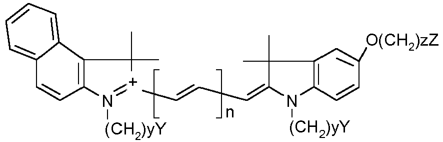
   dans lesquelles chacun de y, z et n, identiques ou différents, est un entier égal à 1, 2, 3, 4, 5, 6, 7 ou 8
   Z représente-COOH ou 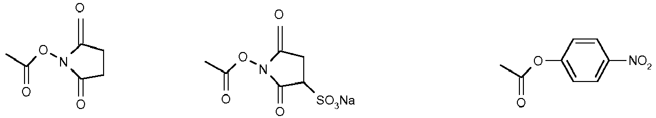
   Y représente SO₃⁻ ou SO₃Na
      en particulier 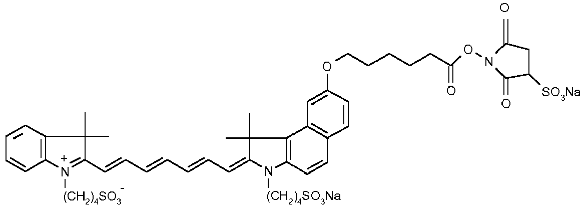 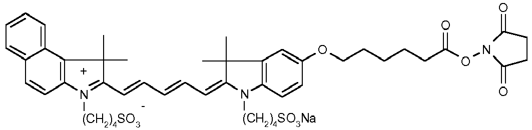
(5) 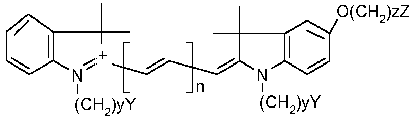 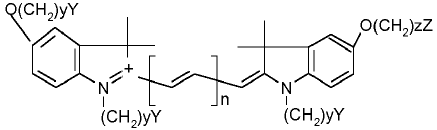
   dans lesquelles chacun de y, z et n, identiques ou différents, est un entier égal à 1, 2, 3, 4, 5, 6, 7 ou 8
   Z représente-COOH ou 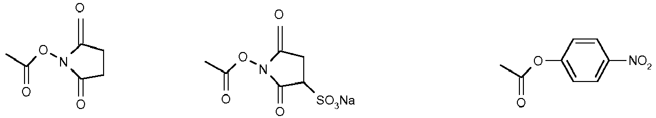
   Y représente SO₃⁻ ou SO₃Na
      en particulier 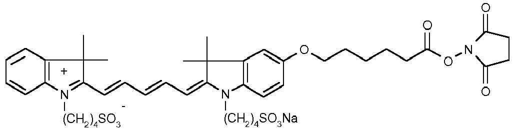
(6) 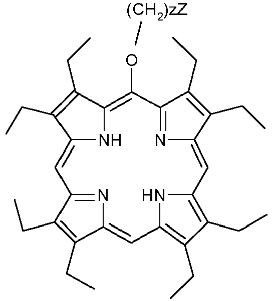 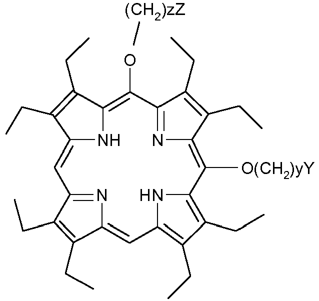
   dans laquelle chacun de y et z, identiques ou différents, est un entier égal à 1, 2, 3, 4, 5, 6, 7 ou 8
      Z représente-COOH ou 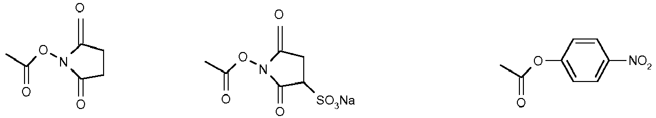
      Y représente SO₃⁻ ou SO₃Na
   en particulier 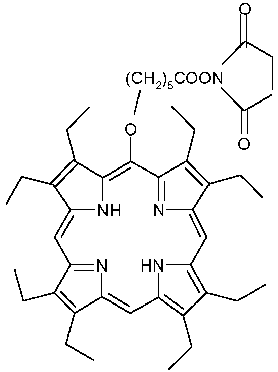
(7) 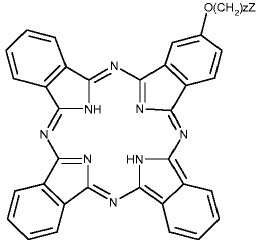 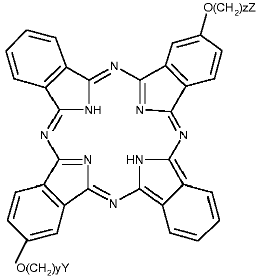
   dans laquelle chacun de y et z, identiques ou différents, est un entier égal à 1, 2, 3, 4, 5, 6, 7 ou 8
   Z représente-COOH ou 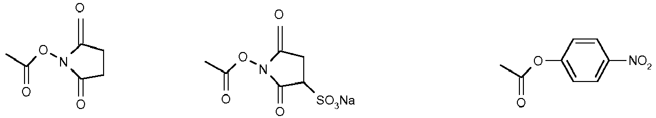
   Y représente SO₃⁻ ou SO₃Na
      en particulier

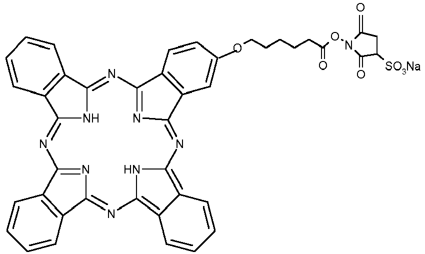

Le procédé de préparation des marqueurs de type carbocyanine de l'invention, et en particulier l'introduction sur un atome de carbone de la structure du colorant, d'au moins un groupe [FONC], permettant de conjuguer lesdits marqueurs à une molécule cible dans un milieu de couplage donné, et éventuellement l'introduction d'au moins un groupe [SOL], constitue le second objet de l'invention.

Le procédé conforme à l'invention grâce auquel il a été possible de synthétiser le marqueur conforme à l'invention, est caractérisé par le fait qu'il comprend une réaction de substitution nucléophile entre :
ou bien :
   Z-A-L et [COLOR']- Nu
ou bien :
   [COLOR"]-L et Z-A-Nu

   - Z et A étant tels que définis à la revendication 1;
   - L représentant un groupe partant ;
   - Nu représentant un groupe nucléophile choisi dans le groupe comprenant -OH, -SH ou -NR₁R₂, R₁ et R₂ étant chacun indépendamment l'un de l'autre un atome d'hydrogène ou un groupe alkyle, linéaire ou ramifié, en C₁-C₃₀, de préférence en C₁-C₁₈, plus préférentiellement en C₁-C₅ ;
   - [COLOR'] et [COLOR"] représentant [COLOR] ou un précurseur ou intermédiaire de synthèse de [COLOR], [COLOR] étant le colorant tel que défini à la revendication 1.

Par "groupe partant", on désigne un groupe qui peut être remplacé par un autre groupe lors d'une réaction de substitution.

Ledit groupe partant peut être notamment un halogène ou un groupe méthanesulfonate, para-toluènesulfonate ou un groupe diazonium. Cette liste n'est pas limitative. Le groupe partant permet donc la fixation de la chaîne portant soit la fonction chimique réactive soit la fonction polaire ou apolaire, sur un atome de carbone de la structure cyclique du colorant ou de son intermédiaire de synthèse.

Selon un mode de réalisation particulier, la réaction de substitution nucléophile peut être réalisée par ouverture d'une molécule cyclique de formule :

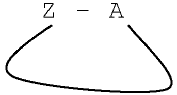

Selon un mode de réalisation particulier, la réaction de substitution nucléophile peut être une réaction de type Williamson, réalisée entre la molécule [COLOR']-Nu et la molécule Z - A - L, en présence d'une base.

Ladite base peut être notamment l'hydroxyde de sodium, le carbonate de potassium, le t-butylate de potassium, l'hydrure de sodium, l'amidure de sodium. Cette liste n'est pas limitative.

L'invention porte également sur les procédés de préparation de [COLOR']-Nu et [COLOR"]-L.
[COLOR']-Nu est obtenu par les réactions successives suivantes :
   1. nitration de [COLOR'];
   2. réduction du produit obtenu en étape 1;
   3. éventuellement : ou bien alkylation du produit obtenu en étape 2, ou bien diazotation du produit obtenu en étape 2 pour l'obtention d'un sel de diazonium puis substitution nucléophile sur cette fonction diazonium.
[COLOR"]-L est quant à lui obtenu par les réactions successives suivantes :
   1'. nitration de [COLOR"];
   2'. réduction du produit obtenu en étape 1';
   3'. diazotation du produit obtenu en étape 2' pour l'obtention d'un sel de diazonium ;
   4'. éventuellement : substitution nucléophile sur la fonction diazonium du produit obtenu en étape 3'.

Selon un mode de réalisation particulier, [COLOR']-Nu et [COLOR"]-L représentent le colorant lui-même, c'est-à-dire [COLOR].

La nitration peut être réalisée par exemple par action de l'acide nitrique, du tétrafluoroborate de nitronium, du nitrate de sodium ou du nitrite de sodium en milieu acide. La réduction du dérivé nitré en amine peut être réalisée par hydrogénation catalytique, par transfert d'hydrogène en présence d'un catalyseur ou par action du chlorure stanneux en milieu acide par exemple. Cette fonction amine peut elle-même être utilisée comme groupe nucléophile ou peut être transformée en autre groupe nucléophile. Dans la mesure où il est préférable d'obtenir un autre groupe nucléophile, celui-ci peut facilement être obtenu par substitution du sel de diazonium correspondant. Dans ce cas, la fonction amine précédente est transformée en sel de diazonium par action du nitrite de sodium en milieu acide. Pour l'obtention d'une fonction hydroxy, le sel de diazonium est hydrolysé en milieu sulfurique dilué par exemple. Pour l'obtention d'une fonction thiol, le sel de diazonium est mis à réagir avec un composé soufré, par exemple un xanthate, puis clivé.

Le moment dans la synthèse où l'introduction du groupe nucléophile Nu (ou du groupe partant L) puis la réaction de substitution avec la molécule Z-A-L (ou Z-A-Nu) seront réalisées, c'est-à-dire le choix du précurseur [COLOR'] (ou [COLOR"]), dépend bien entendu de la nature du colorant utilisé. L'homme du métier saura bien entendu choisir ce moment de façon à limiter les opérations de protection et déprotection de groupes fonctionnels sensibles.

Dans le cas où la synthèse du colorant fonctionnalisé doit débuter avec l'un de ses intermédiaires de synthèse portant le groupe nucléophile Nu, il est recommandé de protéger préalablement ce groupement afin d'éviter les réactions secondaires non désirées.

Ledit groupe nucléophile peut être protégé par un groupement protecteur, par exemple sous forme d'un éther (méthoxy...) pour un groupement hydroxy, d'un thioéther (benzylthio...) pour un groupement thiol, d'un amide ou un groupe de type uréthane par exemple un groupe t-butyloxycarbonyle ou un groupe benzyloxycarbonyle pour une amine.

Après l'obtention du colorant ou de l'un de ses intermédiaires de synthèse, alors que les dernières étapes de synthèse ne risquent plus d'interférer avec la fonction chimique réactive, le groupe nucléophile est déprotégé sous l'action d'un acide, d'une base ou par réduction ou oxydation et est ensuite mis à réagir avec la molécule Z-A-L afin d'introduire la fonction chimique réactive Z.

Selon un mode de réalisation particulier, le procédé de la présente invention, peut comporter la fixation d'un ou plusieurs groupes [SOL] en utilisant une ou plusieurs des étapes de réactions chimiques précédemment définies pour la fixation du groupe [FONC]. Dans ce cas, la réaction de substitution nucléophile est réalisée en utilisant une molécule Z'-A'-L (ou Z'-A'-Nu) dans laquelle:
Z', A', Nu et L sont tels que définis précédemment.
Ainsi, le procédé peut comprendre une réaction de substitution nucléophile entre :
ou bien :
   Z'-A'-L et [COLOR']- Nu
ou bien :
   [COLOR"]-L et Z'-A'-Nu

   - Z' et A', L, Nu, [COLOR'] et [COLOR"] étant tels que définis précédemment;
   - [COLOR']- Nu et [COLOR"]-L étant préparés selon le procédé décrit précédemment.

Le procédé de l'invention permet donc d'obtenir, à partir de colorants facilement accessibles de nouveaux dérivés fonctionnalisés dont les caractéristiques spectrales et les caractéristiques de solubilité sont particulièrement intéressantes pour le marquage des molécules cibles.

Des exemples de schémas réactionnels de préparation de marqueurs à partir de carbocyanines (Schémas Ia et Ib), de phtaléines (Schéma II) et de porphyrines (Schéma III) sont donnés ci-après de façon non limitative.
Dans ces schémas réactionnels, NHS représente la N-hydroxysuccinimide et DCC la dicyclohexylcarbodiimide.

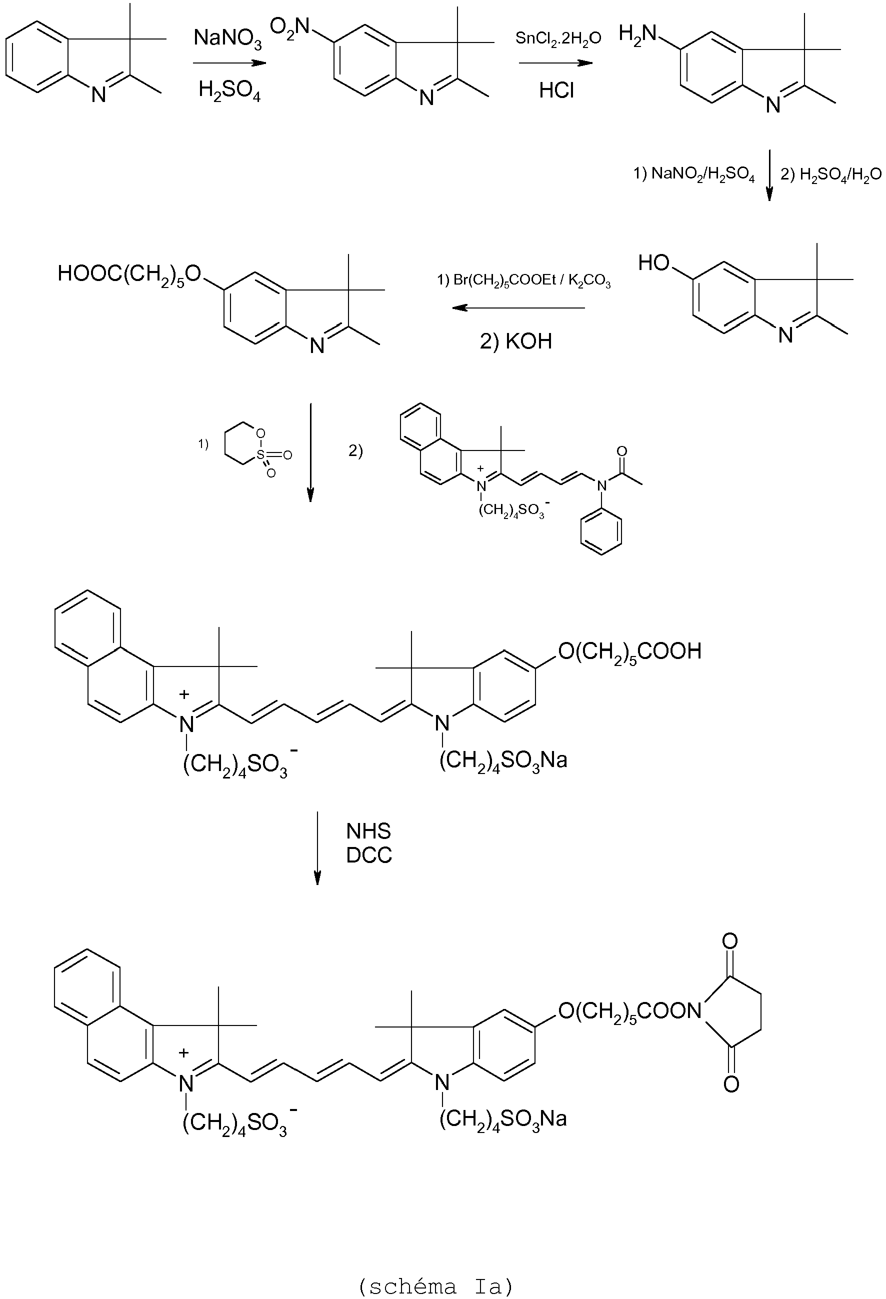

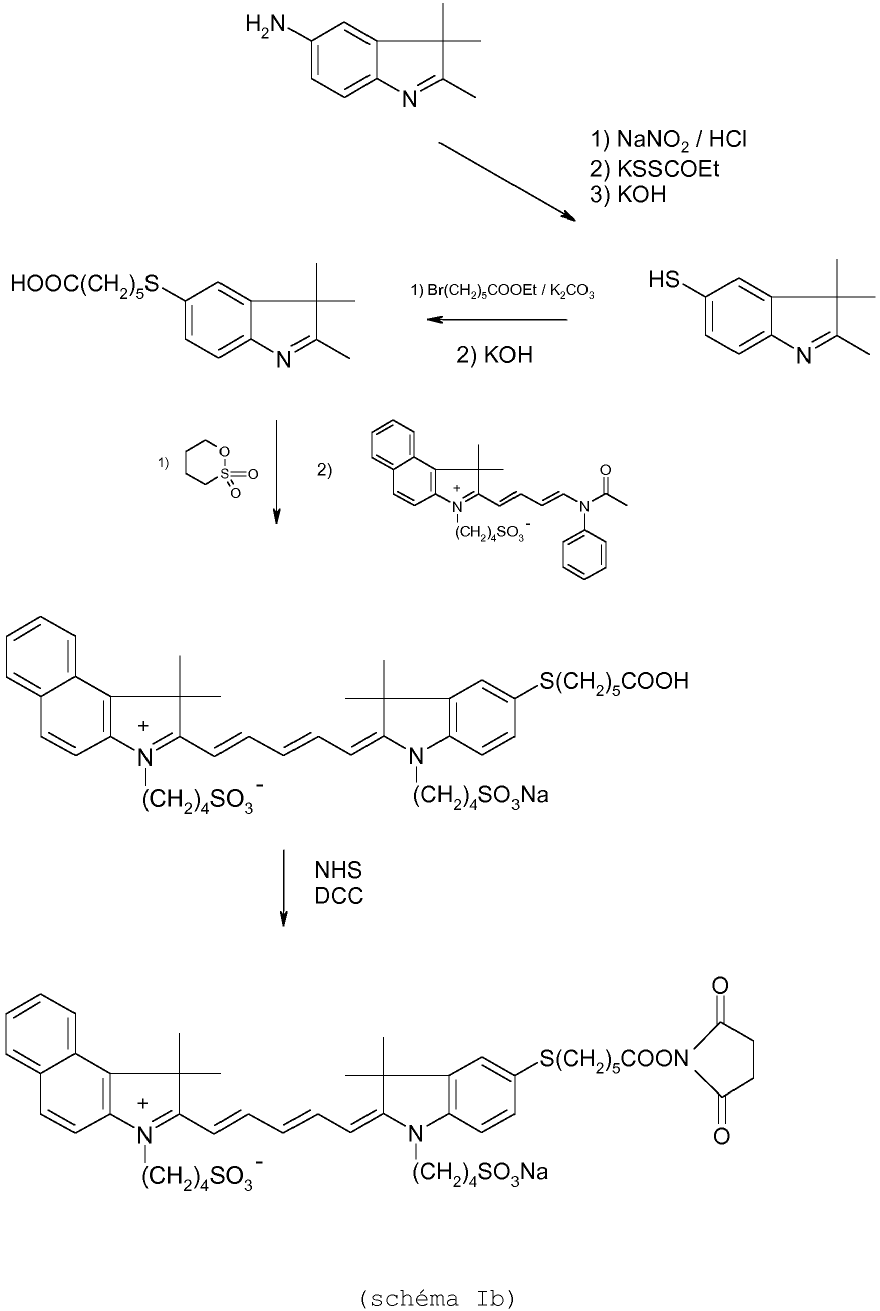

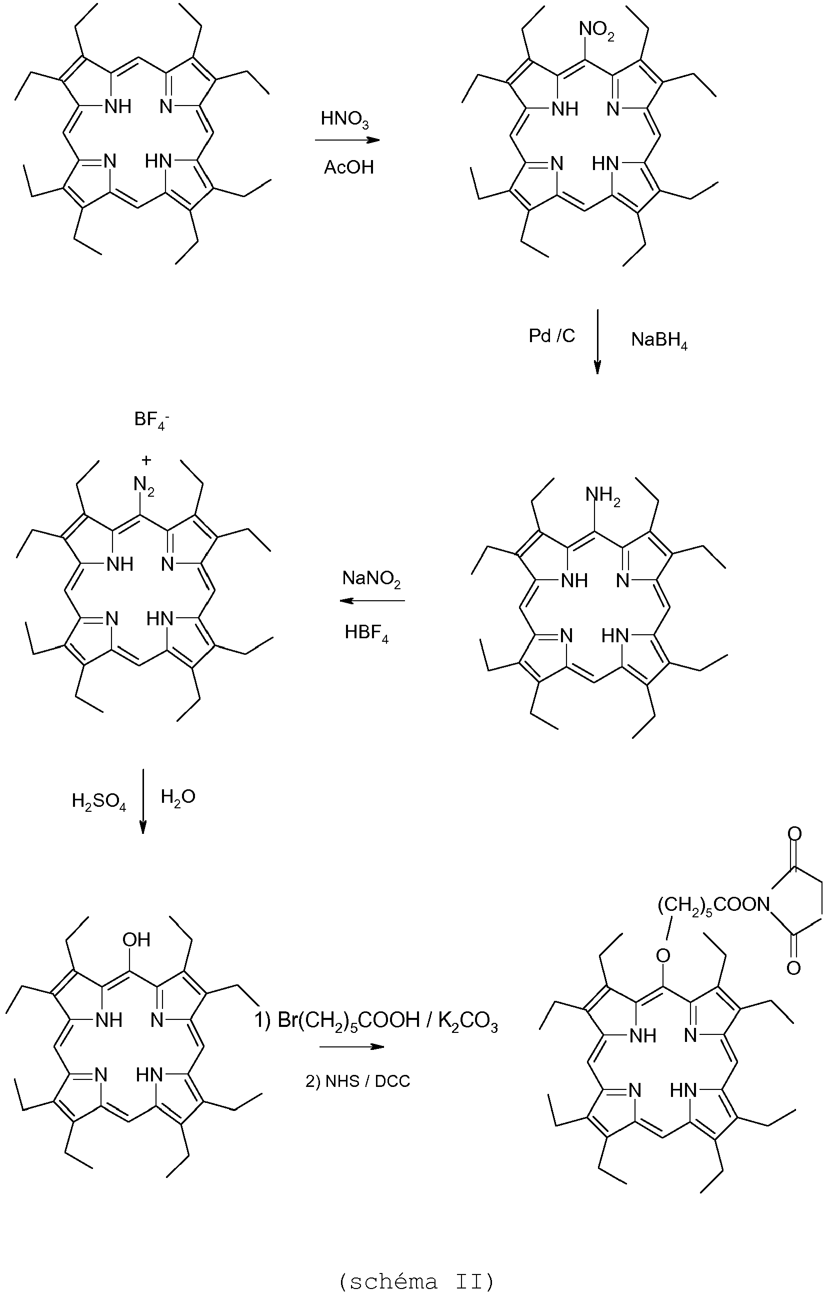

L'application des marqueurs de l'invention, tels que décrits précédemment, à la détection et/ou la quantification de molécules cibles constitue le troisième objet de l'invention.

Comme indiqué précédemment, les marqueurs de l'invention conviennent comme agents pour le marquage de molécules cibles, le marquage étant réalisé par couplage dudit marqueur par liaison covalente ou non covalente avec la molécule cible à doser ou à détecter. Ce couplage peut être réalisé selon des procédés classiques de couplage utilisés dans ce domaine.

Selon un mode de réalisation particulier, la molécule cible est mise à réagir avec 1 à 200 équivalents, plus préférentiellement 3 à 20 équivalents du marqueur. Lorsque la molécule cible est hydrosoluble, la réaction a lieu dans une solution aqueuse de tampon, de préférence à base de phosphate, de carbonate ou de borate, dont le pH est compris entre 7,0 et 11,0. Lorsque la molécule cible est liposoluble, la réaction a lieu dans un solvant organique.

La réaction de couplage peut également être réalisée en présence d'un agent de couplage peptidique, par exemple les réactifs de type carbodiimide comme la DCC (dicyclohexylcarbodiimide), le carbonyldiimidazole, la IDDQ (1-isobutyloxycarbonyl-2-isobutyloxy-1,2-dihydroquinoline), les réactifs de type phosphonium comme le BOP (benzotriazol-1-yloxytris-(diméthylamino)phosphonium hexafluorophosphate), les réactifs de type uronium comme le HBTu (o-benzotriazolyl-tétraméthyluronium hexafluorophosphate) et le TBTu (o-benzotriazolyltétraméthyluronium tétrafluoroborate), les réactifs de Woodward comme le N-éthyl-5-phénylisoxazolium-3'-sulfonate, les réactifs de Curtius (hydrazine et nitrite).

La réaction de couplage est menée si nécessaire en présence d'une base organique et de diméthylsulfoxyde, ou de préférence de diméthylformamide.

La molécule cible marquée est isolée du milieu réactionnel et purifiée par séparation sur gel de silice, par perméation de gel et/ou par ultrafiltration.

L'invention concerne également une méthode de détection d'une molécule biologique ou non biologique comprenant le couplage d'un marqueur conforme à l'invention avec ladite molécule, et la détection proprement dite de ladite molécule couplée au marqueur par spectrométrie d'absorption, spectrométrie de fluorescence, spectrométrie infrarouge, électrophorèse, imagerie médicale dans l'infra-rouge ou le proche infra-rouge (NIRS, Near Infra-Red Spectroscopy). Cette liste n'est pas limitative.

L'invention porte également sur des précurseurs de synthèse des marqueurs de l'invention, tels que décrits à la revendication 10.
Il est aussi décrit des précurseurs de synthèse de marqueurs qui sont des produits nouveaux consistant en des molécules de formule générale : 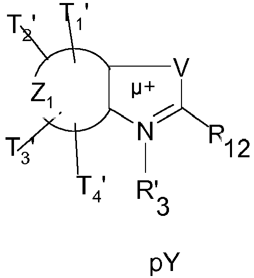 dans laquelle
Z₁, V, p, Y sont tels que définis précédemment,
R'₃ représente un doublet électronique ou représente R₃ tel que défini précédemment ;
µ est un nombre entier égal à 0 ou 1;
R₁₂ représente un groupe alkyle, linéaire ou ramifié, saturé ou insaturé en C₁-C₃₀, de préférence C₁-C₁₈, plus préférentiellement en C₁-C₅, sulfoalkyle, cycloalkyle, aryle, aryloxy, de préférence un groupement méthyle, chacun de T'₁ à T'₄, indépendamment l'un de l'autre, peut avoir la définition donnée précédemment pour T₁ à T₄ ou peut représenter [SOL] ou [FONC], [SOL] et [FONC] étant tels que définis précédemment, et à la condition que l'un au moins parmi T'₁ à T'₄ représente [FONC].

Des exemples, non limitatifs de précurseurs sont donnés ci-après :

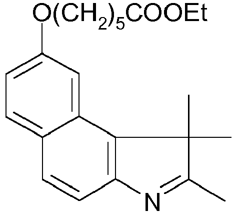

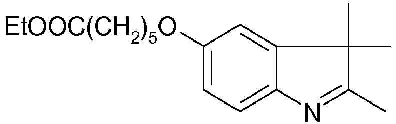

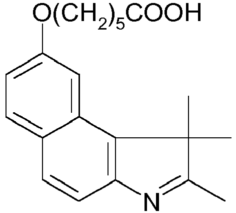

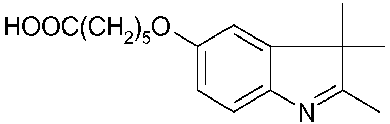

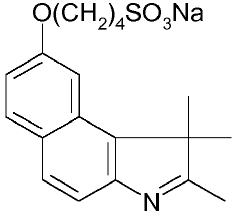

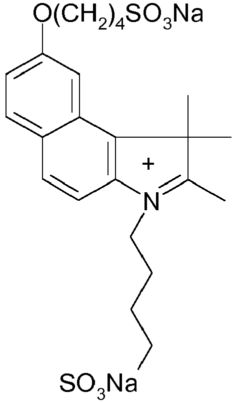

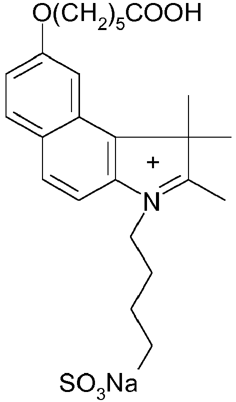

L'invention va être décrite de façon plus détaillée à l'aide des exemples suivants qui ne sont pas limitatifs mais relatifs à des modes de réalisation avantageux.

### EXEMPLES

### EXEMPLE 1

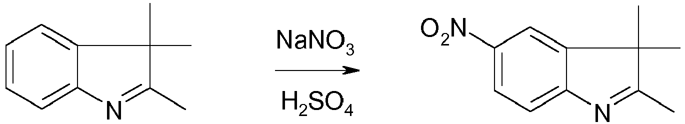

### 5-nitro-2,3,3-triméthyl-(3H)-indole (produit A)

A un mélange de 6,4 g de 2,3,3-triméthyl-(3H)-indole et 50 ml d'acide sulfurique refroidi à 0-5°C est ajoutée, sans dépasser 5°C, une solution de 3,4 g de nitrate de sodium dans 100 ml d'acide sulfurique. Après une heure d'agitation à 0-5°C, le milieu réactionnel est dilué dans 600 ml d'eau et neutralisé par ajout d'hydroxyde de sodium solide. Le précipité formé est filtré et solubilisé dans 200 ml d'acétate d'éthyle. Après lavage à l'eau, séchage sur sulfate de magnésium et évaporation, on obtient 8 g de produit A (Rendement : 97,9%).

### EXEMPLE 2

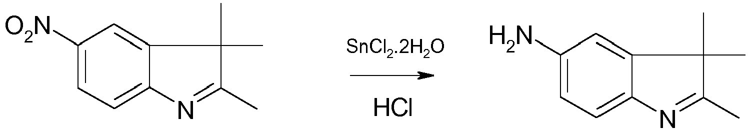

### 5-amino-2,3,3-triméthyl-(3H)-indole (produit B)

Un mélange de 6,6 g de produit A, 43,7 g de chlorure stanneux dihydrate et 215 ml d'acide chlorhydrique est porté à reflux pendant deux heures. Après refroidissement à température ambiante et filtration, le solide recueilli est solubilisé dans 130 ml d'eau. Cette solution est neutralisée par ajout de soude 20%. Le précipité formé est filtré puis lavé à l'eau et séché sous vide en présence d'anhydride phosphorique. On obtient 5,4 g de produit B (Rendement : 96%).

### EXEMPLE 3

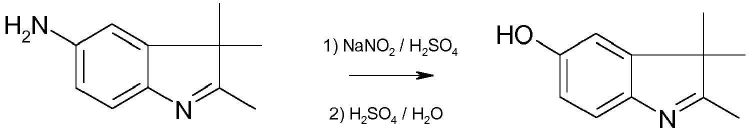

### 5-hydroxy-2,3,3-triméthyl-(3H)-indole (produit C)

Un mélange de 3,5 g de produit B, 5 ml d'acide sulfurique et 25 ml d'eau est refroidi à 0°C. Une solution refroidie à 0-5°C de 1,6 g de nitrite de sodium dans 4 ml d'eau est ajoutée sans dépasser 5°C. Après agitation à 0-5°C pendant 10 mn, le milieu réactionnel est additionné lentement à un mélange de 15 ml d'acide sulfurique et 20 ml d'eau porté à 90°C. Après agitation à cette température pendant une heure puis refroidissement à température ambiante, le milieu réactionnel est neutralisé par ajout de soude 20%. Le précipité formé est filtré puis lavé à l'eau et séché sous vide en présence d'anhydride phosphorique. On obtient 2,6 g de produit C (Rendement : 74,3%).

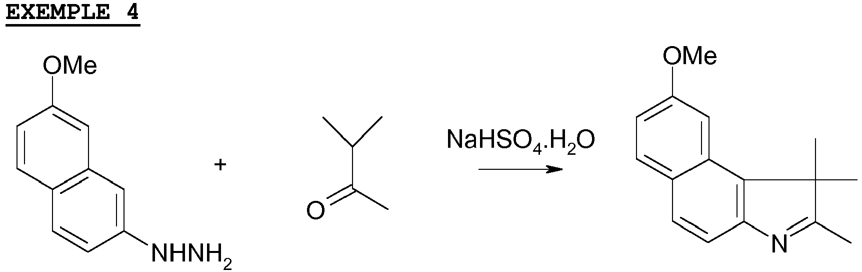

### 5-méthoxy-2,3,3-triméthyl-benz(e)indole (produit D)

Un mélange de 35,4 g de 7-méthoxy-2-naphtylhydrazine et 65 g d'hydrogénosulfate de sodium monohydrate dans 180 ml d'eau est chauffé à 90°C pendant 15 min puis 23 g de 3-méthyl-2-butanone sont additionnés. Le milieu réactionnel est maintenu à 90°C pendant 7 heures, refroidi à température ambiante puis extrait par le dichlorométhane. La phase organique est lavée à l'eau puis évaporée sous vide. Le résidu est purifié par chromatographie sur gel de silice (dichlorométhane/méthanol :10/0,2). On obtient 28,9 g de produit D (Rendement : 64,2%).

### EXEMPLE 5

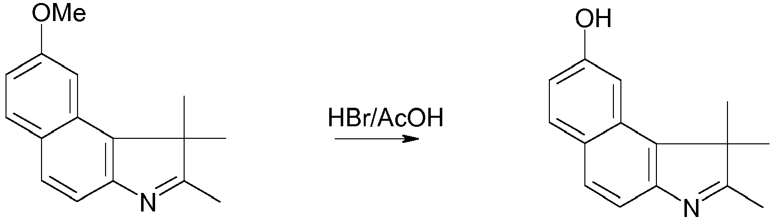

### 5-hydroxy-2,3,3-triméthyl-benz(e)indole (produit E)

8,2 g de produit D sont introduits dans un tricol de 250 ml. 82 ml de HBr (33% dans l'acide acétique) sont ajoutés.

Le mélange est maintenu à 70°C pendant 7 heures puis refroidi à température ambiante. 2,5 litres d'eau puis 120 ml de soude 5M sont ajoutés au milieu réactionnel. Le précipité formé est filtré puis lavé à l'eau et séché sous vide en présence de P₂O₅. On obtient 6,8 g de produit E (Rendement : 88,7%).

### EXEMPLE 6

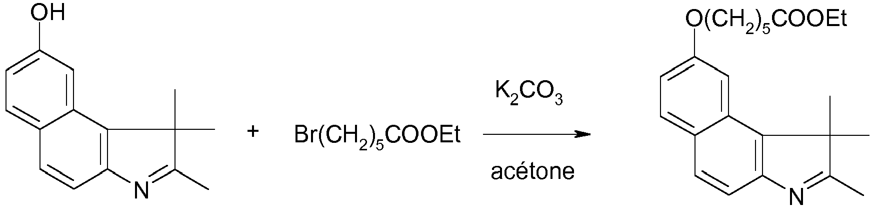

### 5-[(5-carbethoxypentyl)oxy]-2,3,3-trimethyl-benz(e)indole (produit F)

Un mélange de 20 g de produit E, 21,7 g de 6-bromohexanoate d'éthyle et 13,5 g de carbonate de potassium dans 120 ml d'acétone est porté à reflux pendant 8 heures puis refroidi à température ambiante. Le milieu réactionnel est filtré puis évaporé sous vide. Le résidu est dissout dans 400 ml d'éther éthylique et lavé trois fois à l'eau. Après évaporation et élimination de l'excès de 6-bromohexanoate d'éthyle par distillation sous vide, on obtient 32,5 g de produit F (Rendement : 100%) sous forme d'une huile brune.

### EXEMPLE 7

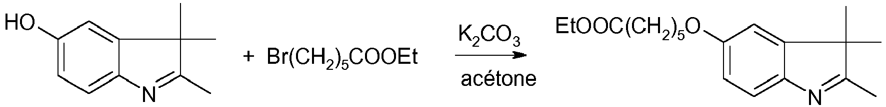

### 5-[(5-carbethoxypentyl)oxy]-2,3,3-trimethyl-(3H)-indole (produit G)

Un mélange de 23,1 g de produit C, 32,4 g de 6-bromohexanoate d'éthyle et 20,1 g de carbonate de potassium dans 180 ml d'acétone est porté à reflux pendant 8 heures puis refroidi à température ambiante. Le milieu réactionnel est filtré puis évaporé sous vide. Le résidu est dissout dans 600 ml d'éther éthylique et lavé trois fois à l'eau. Après évaporation et élimination de l'excès de 6-bromohexanoate d'éthyle par distillation sous vide, on obtient 41,8 g de produit G (Rendement : 100%) sous forme d'une huile brune.

### EXEMPLE 8

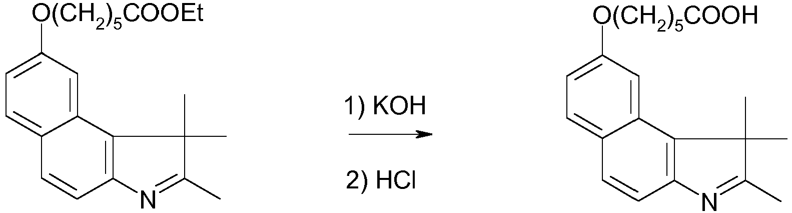

### 5-[(5-carboxypentyl)oxy]-2,3,3-trimethyl-benz(e)indole (produit H)

11,6 g de produit F dans 100 ml de KOH 1M sont chauffés à 80°C pendant 30 mn. Après refroidissement à température ambiante, le milieu réactionnel est neutralisé par ajout d'acide chlorhydrique 1M. Le précipité formé est filtré puis lavé à l'eau et séché sous vide en présence de P₂O₅. On obtient 9 g de produit H (Rendement : 84,2%).

### EXEMPLE 9

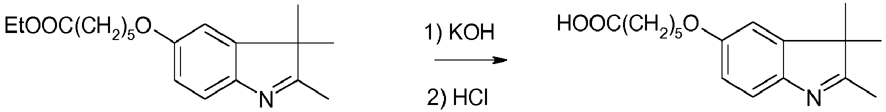

### 5-[(5-carboxypentyl)oxy]-2,3,3-trimethyl-(3H)-indole (produit I)

4,2 g de produit G dans 45 ml de KOH 1M sont chauffés à 80°C pendant 30 mn. Après refroidissement à température ambiante, le milieu réactionnel est neutralisé par ajout d'acide chlorhydrique 1M. Le précipité formé est filtré puis lavé à l'eau et séché sous vide en présence de P₂O₅. On obtient 2,5 g de produit I (Rendement : 66,5%).

### EXEMPLE 10

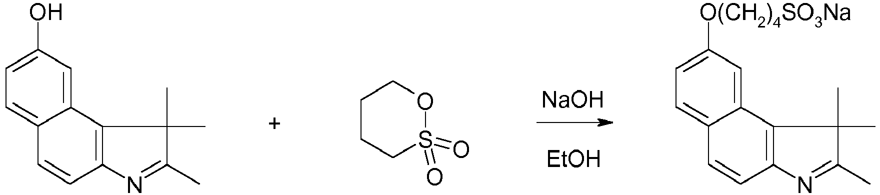

### 5-[(4-sulfobutyl)oxy]-2,3,3-trimethyl-benz(e)indole, sel de sodium (produit J)

Un mélange de 1,3 g de produit E, 0,9 g de 1,4-butane sultone et 0,3 g d'hydroxyde de sodium dans 10 ml d'éthanol est porté à reflux pendant deux heures. Après refroidissement à température ambiante, le milieu réactionnel est additionné à 100 ml d'acétone sous agitation. Le précipité formé est filtré puis lavé à l'acétone et séché sous vide. On obtient 1,8 g de produit J très hygroscopique (Rendement : 81,2%).

### EXEMPLE 11

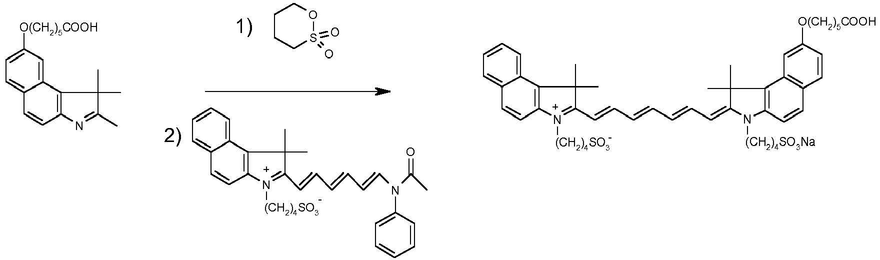

### 2-[7-[8-(5-carboxypentyloxy)-1,3-dihydro-1,1-diméthyl-3-(4-sulfobutyl)-benz(e)indol-2-ylidène]-1,3,5-heptatrienyl]-1,1-dimethyl-3-(4-sulfobutyl)-1H-benz(e)indolium, sel interne, sel de sodium (produit K)

Un mélange de 10,2 g de produit H et 32,7 g de 1,4-butane sultone est chauffé à 115°C pendant 16 heures puis refroidi à température ambiante. 120 ml de toluène sont ajoutés, puis le milieu est filtré pour récupérer la fraction insoluble. Le précipité est rincé à l'acétone et séché sous vide. 13,3 g de ce solide est mis à réagir avec 15,2 g de 2-(6-acétanilido-1,3,5-hexatrienyl)-3,3-dimethyl-1-(4-sulfobutyl)-benz(e)indolium, sel interne, dans 90 ml d'éthanol. 2,9 g de triéthylamine sont ajoutés progressivement et le mélange est porté à reflux pendant 5 minutes puis refroidi à température ambiante. 3,8 g d'acétate de sodium trihydrate sont ajoutés et le mélange est agité pendant 10 minutes. Le précipité formé est filtré puis lavé à l'acétone et séché sous vide. On obtient 18,5 g de produit K (Rendement : 72,8%).

### EXEMPLE 12

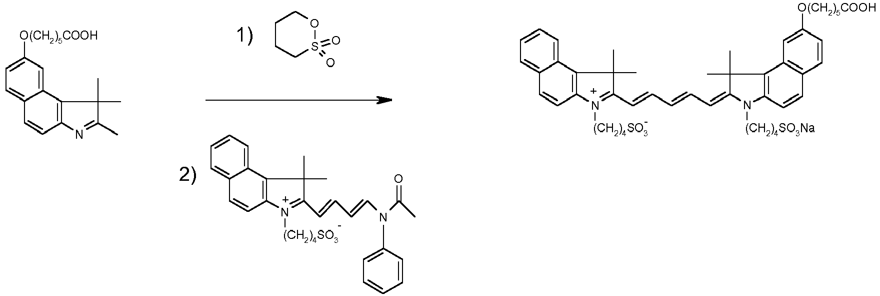

### 2-[5-[8-(5-carboxypentyloxy)-1,3-dihydro-1,1-diméthyl-3-(4-sulfobutyl)-benz(e)indol-2-ylidène]-1,3-pentadienyl]-1,1-dimethyl-3-(4-sulfobutyl)-1H-benz(e)indolium sel interne, sel de sodium (produit L)

Un mélange de 8,1 g de produit H et 26,2 g de 1,4-butane sultone est chauffé à 120°C pendant 12 heures puis refroidi à température ambiante. 80 ml de toluène sont ajoutés, puis le milieu est filtré pour récupérer la fraction insoluble. Le précipité est rincé à l'acétone et séché sous vide. 10,6 g de ce solide est mis à réagir avec 11,5 g de 2-(4-acétanilido-1,3-butadienyl)-3,3-dimethyl-1-(4-sulfobutyl)-benz(e)indolium, sel interne, dans 75 ml d'éthanol. 2,2 g de triéthylamine sont ajoutés progressivement et le mélange est porté à reflux pendant 5 minutes puis refroidi à température ambiante. 3 g d'acétate de sodium trihydrate sont ajoutés et le mélange est agité pendant 10 minutes. Le précipité formé est filtré puis lavé à l'acétone et séché sous vide. On obtient 15,2 g de produit L (Rendement : 75,2%).

### EXEMPLE 13

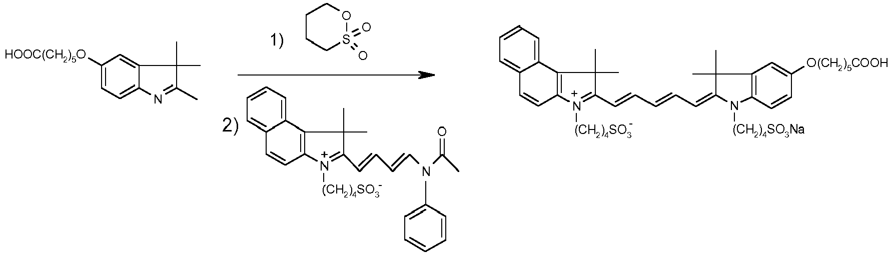

### 2-[5-[6-(5-carboxypentyloxy)-1,3-dihydro-1,1-diméthyl-3-(4-sulfobutyl)-indol-2-ylidène]-1,3-pentadienyl]-1,1-dimethyl-3-(4-sulfobutyl)-1H-benz(e)indolium, sel interne, sel de sodium (produit M)

Un mélange de 10,8 g de produit I et 40,8 g de 1,4-butane sultone est chauffé à 130°C pendant 8 heures puis refroidi à température ambiante. 80 ml de toluène sont ajoutés, puis le milieu est filtré pour récupérer la fraction insoluble. Le précipité est rincé à l'acétone et séché sous vide. 13,4 g de ce solide est mis à réagir avec 16,3 g du sel interne de 2-(4-acétanilido-1,3-butadienyl)-3,3-dimethyl-1-(4-sulfobutyl)-benz(e)indolium dans 175 ml d'éthanol. 3,2 g de triéthylamine sont ajoutés progressivement et le mélange est porté à reflux pendant 5 minutes puis refroidi à température ambiante. 4,3 g d'acétate de sodium trihydrate sont ajoutés et le mélange est agité pendant 10 minutes. Le précipité formé est filtré puis lavé à l'acétone et séché sous vide. On obtient 18,2 g de produit M (Rendement : 69,6%).

### EXEMPLE 14

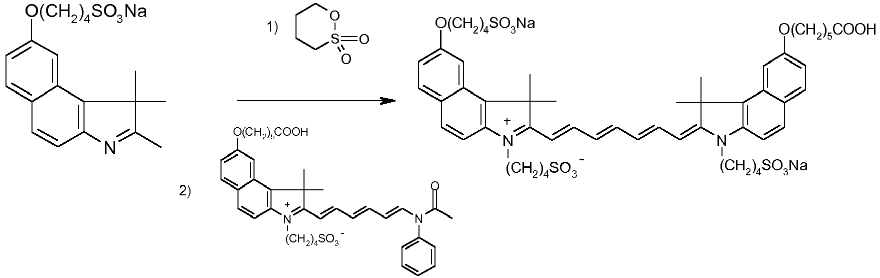

### 2-[7-[8-(5-carboxypentyloxy)-1,3-dihydro-1,1-diméthyl-3-(4-sulfobutyl)-benz(e)indol-2-ylidène]-1,3,5-heptatrienyl]-1,1-dimethyl-3-(4-sulfobutyl)-8-(4-sulfobutyloxy)-1H-benz(e)indolium, sel interne, sel de sodium (produit N)

Un mélange de 0,6 g de produit J et 1,7 g de 1,4-butane sultone est chauffé à 120°C pendant 16 heures puis refroidi à température ambiante. 5 ml de toluène sont ajoutés, puis le milieu est filtré pour récupérer la fraction insoluble. Le précipité est rincé à l'acétone et séché sous vide. 0,7 g de ce solide est mis à réagir avec 0,9 g de 2-(6-acétanilido-1,3,5-hexatrienyl)-5-(5-carboxypentyloxy)-3,3-dimethyl-1-(4-sulfobutyl)-benz(e)indolium, sel interne, (obtenu à partir du sel interne de 5-(5-carboxypentyloxy)-1-(4-sulfobutyl)-2,3,3-trimethyl-benz(e)indolium, dont la préparation est décrite dans les exemples 11 et 12, et de chlorhydrate de dianilide de l'aldéhyde glutaconique) dans 10 ml d'éthanol. 0,14 g de triéthylamine sont ajoutés progressivement et le mélange est porté à reflux pendant 5 minutes puis refroidi à température ambiante. 0,18 g d'acétate de sodium trihydrate sont ajoutés et le mélange est agité pendant 10 minutes. Le précipité formé est filtré puis lavé à l'acétone et séché sous vide. On obtient 1,1 g de produit N (Rendement : 75,5%).

### EXEMPLE 15

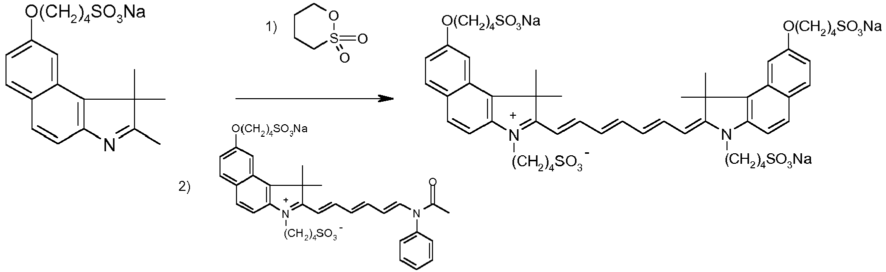

### 2-[7-[8-(4-sulfobutyloxy)-1,3-dihydro-1,1-diméthyl-3-(4-sulfobutyl)-benz(e)indol-2-ylidène]-1,3,5-heptatrienyl]-1,1-dimethyl-3-(4-sulfobutyl)-8-(4-sulfobutyloxy)-1H-benz(e)indolium, sel interne, sel trisodique (produit O)

Un mélange de 0,6 g de produit J et 1,7 g de 1,4-butane sultone est chauffé à 120°C pendant 16 heures puis refroidi à température ambiante. 5 ml de toluène sont ajoutés, puis le milieu est filtré pour récupérer la fraction insoluble. Le précipité est rincé à l'acétone et séché sous vide. 0,7 g de ce solide est mis à réagir avec 0,97 g de 2-(6-acétanilido-1,3,5-hexatrienyl)-3,3-dimethyl-1-(4-sulfobutyl)-5-(4-sulfobutyloxy)-benz(e)indolium, sel interne, sel de sodium, (obtenu à partir de 1-(4-sulfobutyl)-5-(4-sulfobutyloxy)-2,3,3-trimethyl-benz(e)indolium, sel interne, sel de sodium, dont la préparation est décrite précédemment, et de chlorhydrate de dianilide de l'aldéhyde glutaconique) dans 10 ml d'éthanol. 0,14 g de triéthylamine sont ajoutés progressivement et le mélange est porté à reflux pendant 5 minutes puis refroidi à température ambiante. 0,18 g d'acétate de sodium trihydrate sont ajoutés et le mélange est agité pendant 10 minutes. Le précipité formé est filtré puis lavé à l'acétone et séché sous vide. On obtient 1,2 g de produit O (Rendement : 79,1%).

### EXEMPLE 16

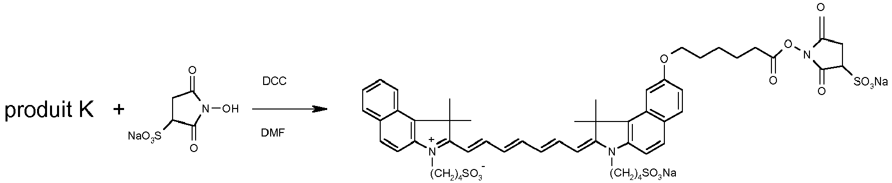

### Sulfosuccinimidyl ester du produit K (produit P)

0,09 g de produit K sont solubilisés dans 5 ml de DMF. 0,11 g de 1-hydroxy-3-succinimide-sulfonate de sodium et 25 mg de DiCyclohexylCarbodiimide sont ajoutés. Le milieu réactionnel est maintenu sous agitation à température ambiante pendant 24 heures puis filtré. 100 ml d'éther éthylique sont additionnés au filtrat. Le précipité formé est filtré et séché sous vide. On obtient 105 mg de produit P (Rendement : 95,4%).

### EXEMPLE 17

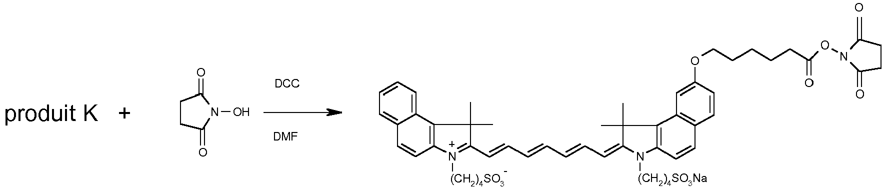

### Succinimidyl ester du produit K (produit Q)

1,08 g de produit K sont solubilisés dans 50 ml de DMF. 0,69 g de N-hydroxysuccinimide et 0,31 g de DiCyclohexylCarbodiimide sont ajoutés. Le milieu réactionnel est maintenu sous agitation à température ambiante pendant 24 heures puis filtré. 250 ml d'éther éthylique sont additionnés au filtrat. Le précipité formé est filtré et séché sous vide. On obtient 1,1 g de produit Q (Rendement : 91,7%).

### EXEMPLE 18

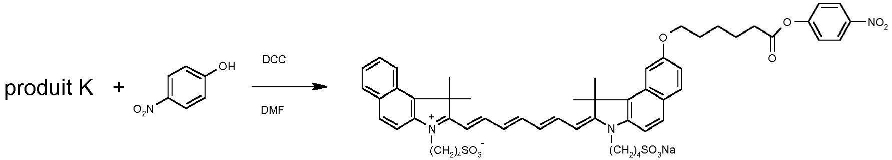

### p-nitrophenyl ester du produit K (produit R)

90 mg de produit K et 17 mg de p-nitrophénol sont solubilisés dans 2 ml de DMF. 25 mg de DiCyclohexylCarbodiimide sont ajoutés. Le milieu réactionnel est maintenu sous agitation à température ambiante pendant 24 heures puis filtré. 100 ml d'éther éthylique sont additionnés au filtrat. Après filtration et séchage sous vide on obtient 101 mg de produit R (Rendement : 98%).

### EXEMPLE 19

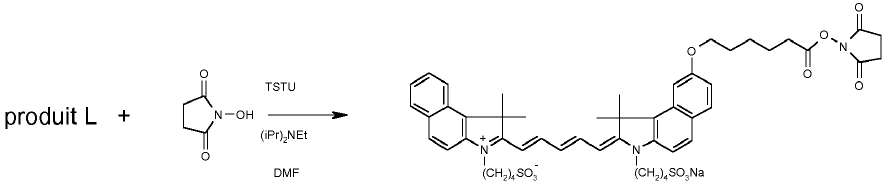

### Succinimidyl ester du produit L (produit S)

0,24 g de produit L et 0,12 g de TSTU (N,N,N',N'-tetramethyl-O-(N-succinimidyl)uronium tetrafluoroborate) sont solubilisés dans 5 ml de DMF. Après 10 minutes d'agitation, 64 mg de diisopropylethylamine sont additionnés. Après 2 heures d'agitation, 100 ml d'éther éthylique sont additionnés. Le précipité formé est filtré, lavé à l'éther éthylique et séché sous vide. On obtient 0,25 g de produit S (Rendement : 92,6%).

### EXEMPLE 20

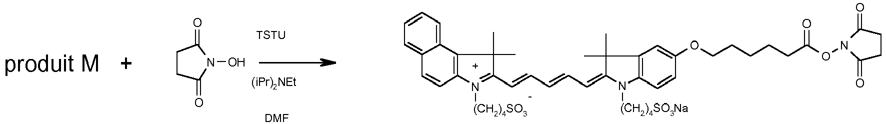

### Succinimidyl ester du produit M (produit T)

83 mg de produit M et 40 mg de TSTU sont solubilisés dans 5 ml de DMF. Après 10 minutes d'agitation, 25 mg de diisopropylethylamine sont additionnés. Après 2 heures d'agitation, 100 ml d'éther éthylique sont additionnés. Le précipité formé est filtré, lavé à l'éther éthylique et séché sous vide. On obtient 90 mg de produit T (Rendement : 96,8%).

### EXEMPLE 21

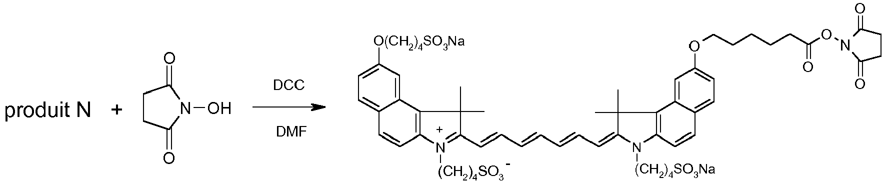

### Succinimidyl ester du produit N (produit U)

1,08 g de produit N sont solubilisés dans 50 ml de DMF. 0,57 g de N-hydroxysuccinimide et 0,26 g de DiCyclohexylCarbodiimide sont ajoutés. Le milieu réactionnel est maintenu sous agitation à température ambiante pendant 24 heures puis filtré. 250 ml d'éther éthylique sont additionnés au filtrat. Le précipité formé est filtré et séché sous vide. On obtient 0,99 g de produit U (Rendement : 84,2%).

### EXEMPLE 22

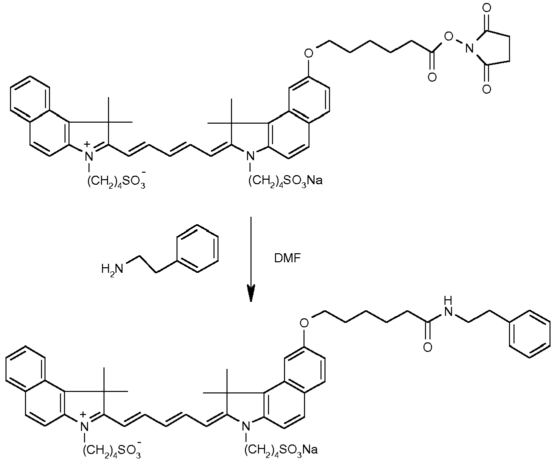

### Phenethyl amide du produit L (produit V)

93 mg de produit S et 39 mg de phenethylamine sont solubilisés dans 5 ml de DMF. Après 45 minutes d'agitation, 60 ml d'éther éthylique sont additionnés. Le précipité formé est filtré, lavé à l'éther éthylique et séché sous vide. On obtient 57 mg de produit V (Rendement : 61,3%).

### EXEMPLE 23

### Marquage de protéines avec le produit S

A une solution de 1 mg de protéine (poids moléculaire 150 kDa) dans 0,5 ml de solution tampon phosphate (0,1 M) on ajoute 1 ml de tampon carbonate-bicarbonate de sodium (0,1 M, pH 9,3) contenant 10% de diméthylformamide, puis 25 µl d'une solution de 9,1 mg de produit S dans 2 ml de diméthylformamide sont additionnés goutte-à-goutte. Le mélange est agité pendant 30 minutes à température ambiante. 200 µl de ce mélange sont élués en fractions de 0,5 ml avec du tampon phosphate (0,1 M, pH 7,4, 10% de diméthylformamide) sur une colonne Sephadex G25/PD-10 (Amersham Biosciences) préalablement équilibrée avec 25 ml du même tampon. Le colorant couplé à la protéine est séparé du colorant non couplé par perméation de gel : la première bande colorée (fractions 7 à 9) correspond au colorant couplé et la deuxième bande colorée (fractions 14 à 17) correspond au colorant libre. La lecture des densités optiques à 685 nm indique un taux de substitution moyen de 4,7 molécules de colorant par molécule d'anticorps. Le conjugué est conservé à 4°C à l'abri de la lumière.

## Revendications

1. Marqueur consistant en un colorant auquel est lié de façon covalente par un ou plusieurs carbones de sa structure chimique :
- un ou plusieurs groupe(s) [FONC], et
- éventuellement un ou plusieurs groupe(s) [SOL]
ledit marqueur présentant la formule générale (7) suivante : 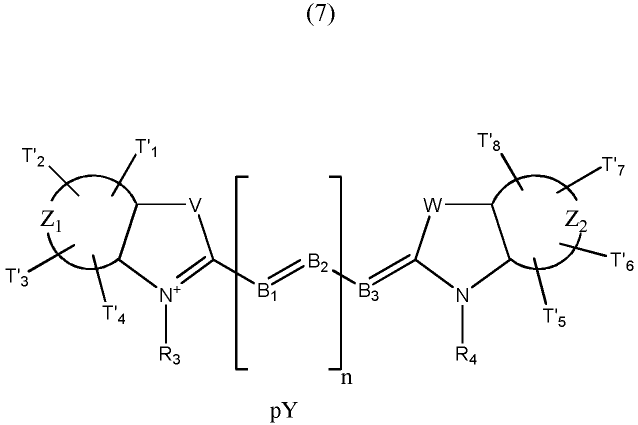
dans laquelle
- Z₁ et Z₂ représentent chacun indépendamment l'un de l'autre les atomes nécessaires pour compléter un noyau benzindole ou naphtindole ;
- V et W sont chacun indépendamment l'un de l'autre choisis parmi CR₇R₈ où R₇ et R₈ sont chacun indépendamment les uns des autres choisis parmi l'hydrogène et un groupe (CH₂)ₘR₁₀, ou m est un nombre entier de 1 à 18 et R₁₀ est sélectionné parmi hydrogène, amine, ammonium quaternaire, aldéhyde, halogène, cyano, aryl, hétéroaryl, hydroxyl, amide, acide sulfonique et ses sels, -COOH ;
- n est un nombre entier de 1 à 10;
- R₃ et R₄ représentent chacun indépendamment les uns des autres un atome d'hydrogène, un groupe alkyle, linéaire ou ramifié en C₁-C₃₀, cycloalkyle ayant de 1 à 30 atomes de carbone, aryle, aryloxy, nitroalkyle ayant de 1 à 30 atomes de carbone, alkylamine, alkylammonium quaternaire ayant de 1 à 30 atomes de carbone, alkylphosphate ayant de 1 à 30 atomes de carbone, acide alkylsulfonique ayant de 1 à 30 atomes de carbone et ses sels ;
- T'₁ à T'₈ représentent chacun indépendamment les uns des autres :
▪ un atome d'hydrogène, un atome d'halogène, un groupe alkyle linéaire ou ramifié en C₁-C₃₀, sulfoalkyle ayant de 1 à 30 atomes de carbone, cycloalkyle ayant de 1 à 30 atomes de carbone, aryle, aryloxy, nitro, amine, ammonium quaternaire, phosphate, acide sulfonique et ses sels, OR₁₁ avec R₁₁ choisi parmi l'hydrogène et un groupe alkyle en C₁-C₃₀, COOR₁₁ ou CONHR₁₁ avec R₁₁ tel que défini précédemment ;
▪ [SOL] ; ou
▪ [FONC],
à la condition que l'un au moins parmi T'₁ à T'₈, représente [FONC],- B₁, B₂, B₃ représentent chacun indépendamment les uns des autres un groupement méthine (=CH-);
- Y représente un contre-ion choisi parmi les ions halogénure, p-toluènesulfonate, méthanesulfonate, trifluorométhane-sulfonate, perchlorate, acétate, sodium, potassium, calcium, magnésium, lithium, ammonium et trialkylammonium ;
- p est un nombre entier de 0 à 8 nécessaire à la neutralité de la molécule;
[FONC] est choisi parmi -X-(CH₂)ᵣ-COOSu, -X-(CH₂)ᵣ-COOSuSO₃Nₐ, -S-(CH₂)ᵣ-COOH, -O-(CH₂)ₛ-COOH, -X-(CH₂)ᵣ-COO-C₆H₄-NO₂, -X-(C₆H₄)-(CH₂)ᵣ-COOSu,-X-(CH₂)ᵣ-NHCOCH₂I,- -(CH₂)ᵣ-NCS, -X-(CH₂)ᵣ-C₆H₄CH(CH₃)-COOSu et -X-(CH₂)ᵣ-OP[N(iPr)₂][CH₂CH₂CN], X étant un atome d'oxygène ou un atome de soufre, r étant un nombre entier de 1 à 18, s étant un nombre entier de 3 à 18 et Su représentant le groupement succinimidyle ;
[SOL] représentant chacun indépendamment un groupe X'-A'-Z', dans lequel :
- X' est un atome d'oxygène ou un atome de soufre,
- A' est un groupe alkylène ou un groupe alkylène-arylène;
Z' est choisi dans le groupe constitué des groupes acide sulfonique et ses sels, ammonium quaternaire, carbohydrate, glycol, hydroxyle, nitro, phosphate, alkyle linéaire ou ramifié ayant de 1 à 30 atomes de carbone, cycloalkyle ayant de 3 à 14 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, aryle, aryloxy, , aryl-(alkyle ayant de 1 à 30 atomes de carbone) et halogénoaryle ;
**caractérisé par le fait que** le groupe alkylène est une chaîne hydrocarbonée, cyclique, linéaire ou ramifiée, présentant deux liaisons libres, contenant de 1 à 30 atomes de carbone, et **par le fait que** le groupe arylène est un groupement aromatique, présentant deux liaisons libres, contenant un ou plusieurs cycles aromatiques, éventuellement substitué.

2. Marqueur selon la revendication 1, **caractérisé par le fait que** [SOL] est choisi parmi - X-(CH₂)ᵣ-SO₃Na, -X-(CH₂)ᵣ-SO₃H, -X-(CH₂)ᵣ-C₆H₃(NO₂)₂ et -X-(CH₂)ᵣ-CH₃, X étant tel que défini dans la revendication 1 et r étant un nombre entier de 1 à 18.

3. Marqueur selon la revendication 1 ou 2, **caractérisé par le fait qu'**il est choisi dans le groupe comprenant
1) 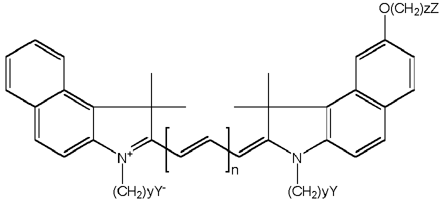 dans laquelle chacun de y et n, identiques ou différents, est un entier égal à 1, 2, 3, 4, 5, 6, 7 ou 8, z représente un entier égal à 3, 4, 5, 6, 7 ou 8, Z représente-COOH ou 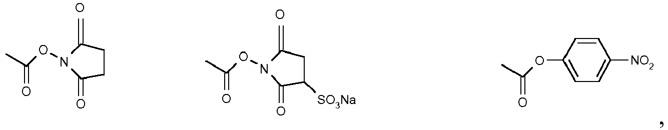 et Y représente SO₃ ou SO₃Na,
2) 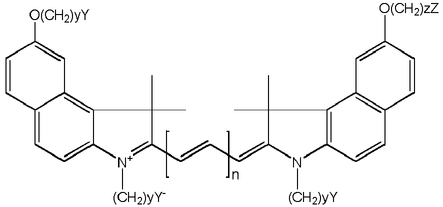 dans laquelle chacun de y et z, identiques ou différents, est un entier égal à 3, 4, 5, 6, 7 ou 8, n est un entier égal à 1, 2, 3, 4, 5, 6, 7 ou 8,
Z représente -COOH ou 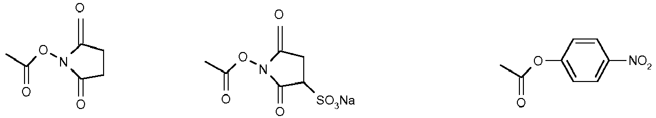 et Y représente SO₃ ou SO₃Na.

4. Marqueur selon la revendication 3, **caractérisé par le fait qu'**il est choisi dans le groupe comprenant
1) 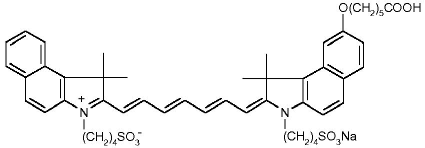 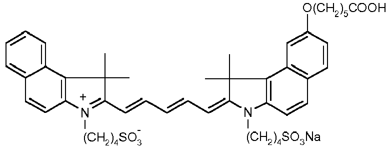 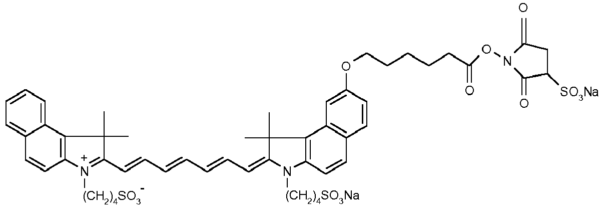 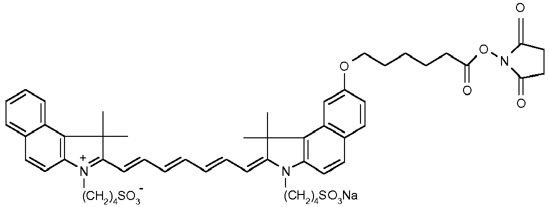 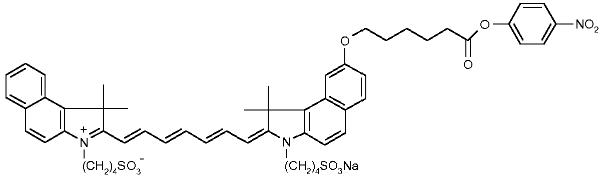 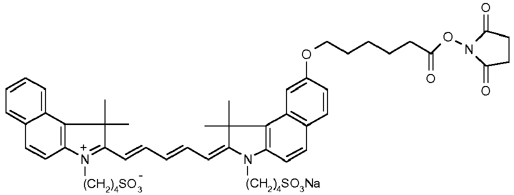
2)

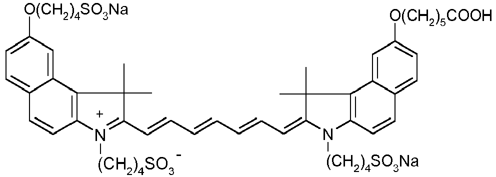

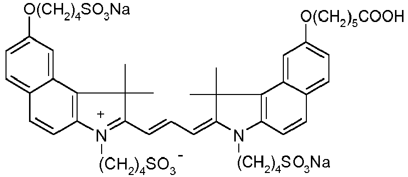

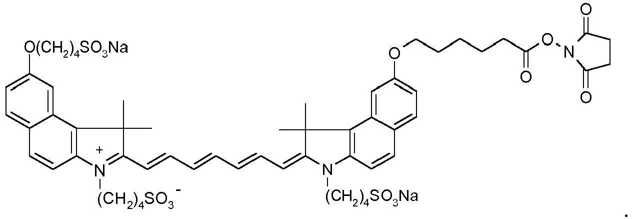

5. Procédé de préparation de marqueurs selon l'une quelconque des revendications 1 à 4, **caractérisé par le fait qu'**il comprend une réaction de substitution nucléophile entre :
ou bien :
Z-A-L et [COLOR']- Nu
ou bien :
[COLOR"]-L et Z-A-Nu
- Z et A étant tels que définis à la revendication 1 ;
- L représentant un groupe partant choisi dans le groupe consistant en un halogène, un groupe méthanesulfonate, para-toluènesulfonate et un groupe diazonium;
- Nu représentant un groupe nucléophile choisi dans le groupe comprenant - OH, -SH et -NR₁R₂, R₁ et R₂ étant chacun indépendamment l'un de l'autre un atome d'hydrogène ou un groupe alkyle linéaire ou ramifié en C₁-C₃₀ ;
[COLOR'] et [COLOR"] représentant le colorant tel que défini dans la revendication 1; 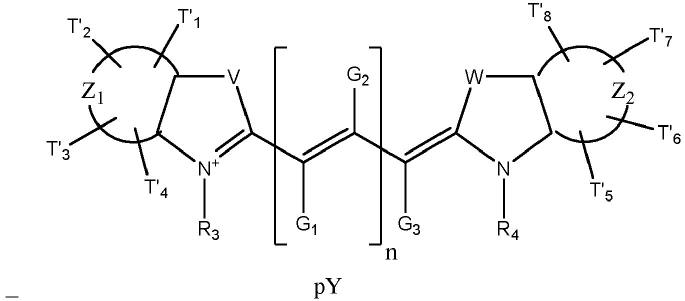
- dans lequel aucun des T'₁ à T'₈_ ne représente [SOL] ou ne représente [FONC] ;
la réaction de substitution nucléophile étant une réaction de Williamson en présence d'une base.

6. Procédé selon la revendication 5, **caractérisé par le fait que** [COLOR']-Nu est obtenu par les réactions successives suivantes :
1. nitration de [COLOR'];
2. réduction du produit obtenu en étape 1 ;
3. éventuellement : ou bien alkylation du produit obtenu en étape 2, ou bien diazotation du produit obtenu en étape 2 pour l'obtention d'un sel de diazonium puis substitution nucléophile sur cette fonction diazonium.

7. Procédé selon la revendication 5 ou 6, **caractérisé par le fait que** [COLOR"]-L est obtenu par les réactions successives suivantes :
1'. nitration de [COLOR"];
2'. réduction du produit obtenu en étape 1';
3'. diazotation du produit obtenu en étape 2' pour l'obtention d'un sel de diazonium ;
4'. éventuellement : substitution nucléophile sur la fonction diazonium du produit obtenu en étape 3'.

8. Procédé selon l'une quelconque des revendications 5 à 7, **caractérisé par le fait qu'**il comprend une réaction de substitution nucléophile entre :
ou bien :
Z'-A'-L et [COLOR']- Nu
ou bien :
[COLOR"]-L et Z'-A'-Nu
- Z' et A' étant tels que définis à la revendication 1 ;
- L, Nu, [COLOR'] et [COLOR"] étant tel que définis dans la revendication 7 ;
- [COLOR']- Nu et [COLOR"]-L étant préparés selon le procédé d'une quelconque des revendications 6 et 7.

9. Procédé de marquage d'une molécule cible avec un marqueur selon l'une quelconque des revendications 1 à 4 ou préparé selon l'une quelconque des revendications 5 à 8.

10. Produit de formule : 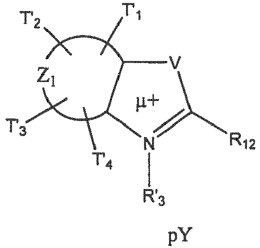 dans laquelle
Z₁, V, p, Y sont tels que définis dans la revendication 1,
R'₃ représente un doublet électronique ou représente R₃ tel que défini dans la revendication 1;
µ est un nombre entier égal à 0 ou 1 ;
R₁₂ représente un groupe alkyle, linéaire ou ramifié, saturé ou insaturé en C₁-C₃₀, sulfoalkyle ayant de 1 à 30 atomes de carbone, cycloalkyle ayant de 1 à 30 atomes de carbone, aryle, aryloxy,
T'₁ à T'₄, ayant la définition donnée à la revendication 1,
à la condition que l'un au moins parmi T'₁ à T'₄, représente [FONC] tel que défini à la revendication 1.

11. Produit selon la revendication 10, **caractérisé en ce qu'**il est choisi dans le groupe constitué de :

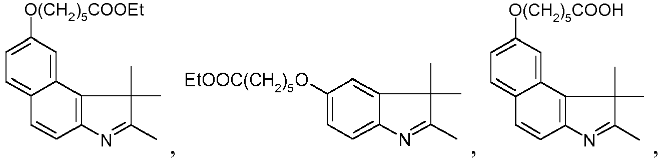

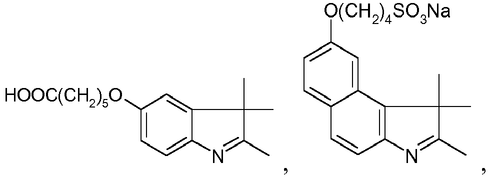

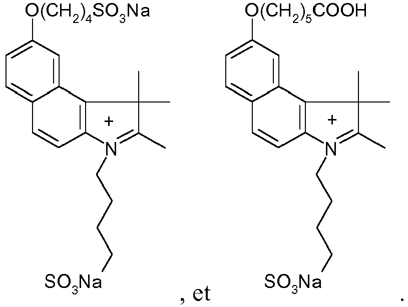

12. Utilisation d'un marqueur selon l'une quelconque des revendications 1 à 4 ou préparé selon l'une des revendications 5 à 8 dans une méthode de marquage de molécules biologiques.

13. Utilisation d'un marqueur selon l'une quelconque des revendications 1 à 4 dans une réaction de couplage en présence d'un agent de couplage peptidique choisi dans le groupe comprenant les réactifs de type carbodiimide comme la DCC (dicyclohexylcarbodiimide), le carbonyldiimidazole, la IDDQ (1-isobutyloxycarbonyl-2-isobutyloxy-1,2-dihydroquinoline) ; les réactifs de type phosphonium comme le BOP (benzotriazol-1-yloxytris-(diméthylamino)phosphonium hexafluorophosphate) ; les réactifs de type uronium comme le HBTu (o-benzotriazolyl-tétraméthyluronium hexafluorophosphate) et le TBTu (o-benzotriazolyltétraméthyluronium tétrafluoroborate) ; les réactifs de Woodward comme le N-éthyl-5-phénylisoxazolium-3'-sulfonate ; et les réactifs de Curtius (hydrazine et nitrite).

14. Utilisation selon la revendication 12 ou 13, **caractérisée par le fait que** la réaction de couplage est réalisée en présence de diméthylformamide.

15. Méthode de détection d'une molécule biologique ou non biologique comprenant le couplage d'un marqueur selon l'une des revendications 1 à 4 avec ladite molécule, et la détection de ladite molécule couplée au marqueur par spectrométrie d'absorption, spectrométrie de fluorescence, spectrométrie infra-rouge, électrophorèse, imagerie médicale dans le proche infrarouge ou l'infrarouge.

## Patentansprüche

1. Marker, der aus einem Farbstoff besteht, bei dem an ein oder mehrere Kohlenstoffatome seiner chemischen Struktur
- eine oder mehrere [FONC] Gruppe(n), und
- gegebenenfalls eine oder mehrere [SOL] Gruppe(n) kovalent gebunden sind,
wobei der Marker durch die folgende allgemeine Formel (7) dargestellt ist: 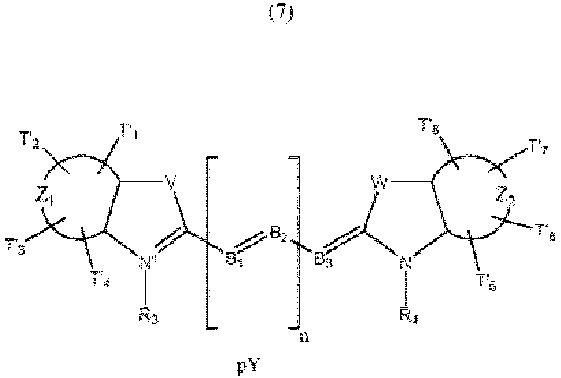
worin
- Z₁ und Z₂ jeweils unabhängig voneinander für die zur Vervollständigung eines Benzindol- oder Naphtindolkerns nötigen Atome steht;
- V und W jeweils unabhängig voneinander aus CR₇R₈ ausgewählt ist, wobei R₇ und R₈ jeweils unabhängig voneinander ausgewählt ist aus Wasserstoff und einer (CH₂)ₘR₁₀ Gruppe, wobei m eine ganze Zahl von 1 bis 18 ist und R₁₀ aus Wasserstoff, einem Aminogruppe, einer quartären Ammoniumgruppe, einer Aldehydgruppe, einer Halogengruppe, einer Cyanogruppe, einer Arylgruppe, einer Heteroarylgruppe, einer Hydroxylgruppe, einer Amidgruppe, einer Sulfonsäuregruppe und ihren Salzen, und -COOH ausgewählt ist;
- R₃ und R₄ jeweils unabhängig voneinander für ein Wasserstoffatom, eine lineare oder verzweigte C₁-C₃₀ Alkylgruppe, eine Cycloalkylgruppe mit 1 bis 30 Kohlenstoffatomen, eine Arylgruppe, eine Aryloxygruppe, eine Nitroalkylgruppe mit 1 bis 30 Kohlenstoffatomen, eine Alkylaminogruppe, eine quartäre Alkylammoniumgruppe mit 1 bis 30 Kohlenstoffatomen, ein Alkylphosphat mit 1 bis 30 Kohlenstoffatomen, eine Alkylsulfonsäure mit 1 bis 30 Kohlenstoffatomen und ihren Salzen steht;
- T'₁ bis T'₈ jeweils unabhängig voneinander für:
▪ ein Wasserstoffatom, ein Halogenatom, eine lineare oder verzweigte C₁-C₃₀ Alkylgruppe, eine Sulfoalkylgruppe mit 1 bis 30 Kohlenstoffatomen, eine Cycloalkylgruppe mit 1 bis 30 Kohlenstoffatomen, eine Arylgruppe, eine Aryloxygruppe, eine Nitrogruppe, eine Aminogruppe, eine quartäre Ammoniumgruppe, eine Phosphatgruppe, eine Sulfonsäuregruppe und ihre Salze, OR₁₁, wobei R₁₁ aus Wasserstoff und einer C₁-C₃₀ Alkylgruppe ausgewählt ist, COOR₁₁ oder CONHR₁₁, wobei R₁₁ wie zuvor definiert ist;
▪ [SOL]; oder
▪ [FONC]
steht,
unter der Voraussetzung, dass mindestens eines von T'₁ bis T'₈ für [FONC] steht,
- B₁, B₂, B₃ jeweils unabhängig voneinander für eine Methingruppe (=CH-) steht;
- Y für ein Gegenion steht, das aus den Halogenid-Ionen, p-Toluolsulfonat, Methansulfonat, Trifluormethansulfonat, Perchlorat, Acetat, Natrium, Kalium, Calcium, Magnesium, Lithium, Ammonium und Trialkylammonium ausgewählt ist;
- p eine für die Neutralität des Moleküls nötige, ganze Zahl von 0 bis 8 ist;
[FONC] aus -X-(CH₂)ᵣ-COOSu, -X-(CH₂)ᵣ-COOSuSO₃Na, -S-(CH₂)ᵣ-COOH, -O-(CH₂)ₛ-COOH, -X-(CH₂ᵣ-COO-C₆H₄-NO₂, ᵣ-COO-C₆H₄-NO₂, -X-(C₆H₄) - (CH₂)ᵣ-COOSu, -X-(CH₂)ᵣ-NHCOCH₂I, -X-(CH₂)ᵣ-NCS, -X-(CH₂)ᵣ-C₆H₄CH (CH₃) -COOSu und -X-(CH₂)ᵣ-OP [N(iPr)₂][CH₂CH₂CN] ausgewählt ist, wobei X ein Sauerstoffatom oder ein Schwefelatom ist, r eine ganze Zahl von 1 bis 18 ist, s eine ganze Zahl von 3 bis 18 ist und Su für eine Succinimidyl-Gruppe steht;
[SOL] jeweils unabhängig für eine -X'-A'-Z' Gruppe steht, worin:
- X' ein Sauerstoff- oder Schwefelatom ist;
- A' eine Alkylengruppe oder eine Alkylen-Arylengruppe ist;
- Z' aus der Gruppe bestehend aus Sulfonsäuregruppen und ihren Salzen, quartären Ammoniumgruppen, Kohlenhydratgruppen, Glykolgruppen, einer Hydroxylgruppe, einer Nitrogruppe, Phosphatgruppen, linearen oder verzweigten Alkylgruppen mit 1 bis 30 Kohlenstoffatomen, Cycloalkylgruppen mit 3 bis 14 Kohlenstoffatomen, Alkyloxygruppen mit 1 bis 30 Kohlenstoffatomen, Halogenalkylgruppen mit 1 bis 30 Kohlenstoffatomen, Hydroxyalkylgruppen mit 1 bis 30 Kohlenstoffatomen, Alkylestergruppen mit 2 bis 40 Kohlenstoffatomen, Arylgruppen, Aryloxygruppen, Aryl-(Alkylgruppen mit 1 bis 30 Kohlenstoffatomen) und Halogenarylgruppen ausgewählt ist;
**dadurch gekennzeichnet, dass** die Alkylengruppe eine cyclische, lineare oder verzweigte Kohlenwasserstoffkette ist, die zwei freie Bindungen aufweist und 1 bis 30 Kohlenstoffatome enthält, und dass die Arylengruppe eine aromatische Gruppe ist, die zwei freie Bindungen aufweist, ein oder mehrere aromatische Ringe enthält und gegebenenfalls substituiert ist.

2. Marker gemäß Anspruch 1, **dadurch gekennzeichnet, dass** [SOL] aus -X-(CH₂)ᵣ-SO₃Na, -X-(CH₂)ᵣ-SO₃H, -X-(CH₂)ᵣ-C₆H₃(NO₂)₂ und -X-(CH₂)ᵣ-CH₃ ausgewählt ist, wobei X wie in Anspruch 1 definiert ist und r eine ganze Zahl von 1 bis 18 ist.

3. Marker gemäß Anspruch 1 oder 2, **dadurch gekennzeichnet, dass** er ausgewählt ist aus der Gruppe, umfassend:
1) 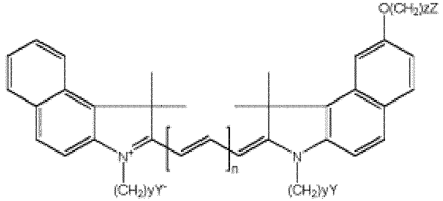
worin jedes y und n, gleich oder verschieden, für eine ganze Zahl gleich 1, 2, 3, 4, 5, 6, 7 oder 8 steht, z für eine ganze Zahl gleich 3, 4, 5, 6, 7, oder 8 steht,
Z für -COOH oder 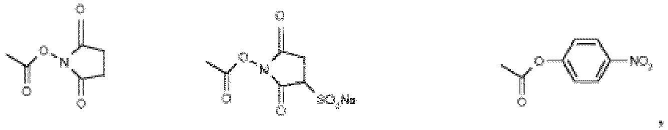
steht, und Y für SO₃ oder SO₃Na steht,
2) 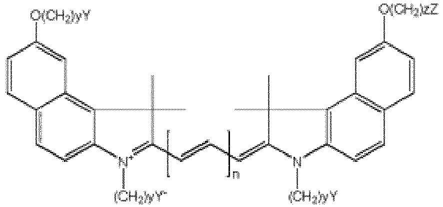 worin jedes y und z, gleich oder verschieden, für eine ganze Zahl gleich 3, 4, 5, 6, 7 oder 8 steht, n für eine ganze Zahl von 1, 2, 3, 4, 5, 6, 7, oder 8 steht, Z für -COOH oder 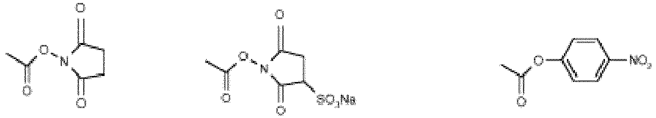 steht, und Y für SO₃ oder SO₃Na steht.

4. Marker gemäß Anspruch 3, **dadurch gekennzeichnet, dass** er ausgewählt ist aus der Gruppe, umfassend:
1) 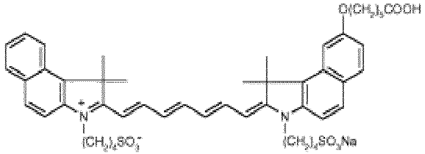 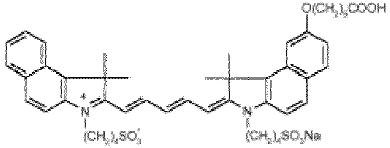 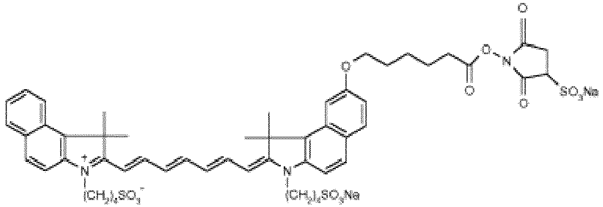 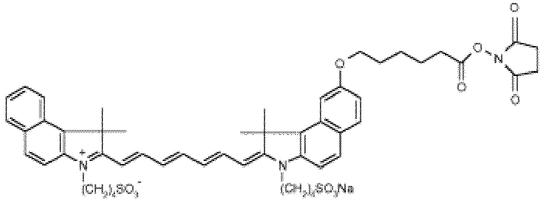 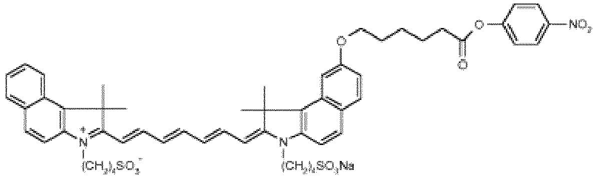 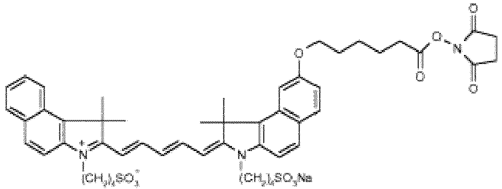
2)

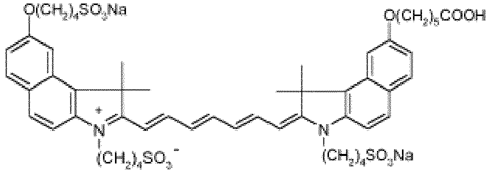

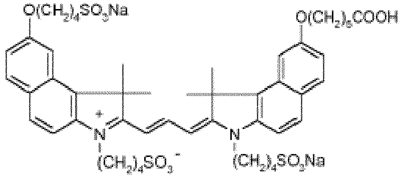

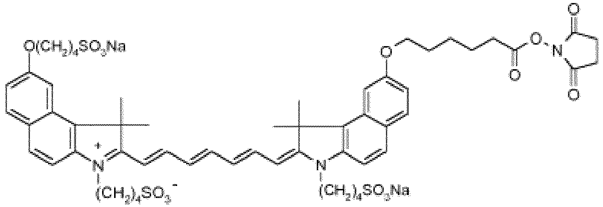

5. Verfahren zur Herstellung von Markern gemäß einem der Ansprüche 1 bis 4, **dadurch gekennzeichnet, dass** es eine nukleophile Substitutionsreaktion entweder zwischen:
Z-A-L und [COLOR']-Nu
oder zwischen:
[COLOR"]-L und Z-A-Nu
umfasst,
- wobei Z und A wie in Anspruch 1 definiert sind;
- wobei L für eine Abgangsgruppe steht, die aus der Gruppe bestehend aus einem Halogen, einer Methansulfonatgruppe, para-Toluolsulfonat und einer Diazoniumgruppe ausgewählt ist;
- wobei Nu für eine nukleophile Gruppe steht, die aus der Gruppe umfassend -OH, -SH, und NR₁R₂, wobei R₁ und R₂ jeweils unabhängig voneinander für ein Wasserstoffatom oder eine lineare oder verzweigte C₁-C₃₀ Alkylgruppe steht ausgewählt ist;
- wobei [COLOR'] und [COLOR"] für den in Anspruch 1 definierten Farbstoff stehen; 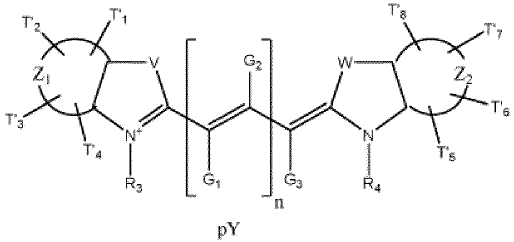
- worin keines von T'₁ bis T'₈ für [SOL] oder [FONC] steht;
wobei die nukleophile Substitutionsreaktion eine Williamson-Reaktion in der Gegenwart einer Base ist.

6. Verfahren gemäß Anspruch 5, **dadurch gekennzeichnet, dass** [COLOR']-Nu durch die folgenden aufeinanderfolgenden Reaktionen erhalten wird:
1. Nitrierung von [COLOR'];
2. Reduktion des in Schritt 1 erhaltenen Produkts;
3. Gegebenenfalls: Entweder Alkylierung des in Schritt 2 erhaltenen Produkts, oder Diazotierung des in Schritt 2 erhaltenen Produkts, um ein Diazoniumsalz zu erhalten und anschließende nukleophile Substitution an der funktionellen Diazoniumgruppe.

7. Verfahren gemäß Anspruch 5 oder 6, **dadurch gekennzeichnet, dass** [COLOR"]-L durch die folgenden aufeinanderfolgenden Reaktionen erhalten wird:
1'. Nitrierung von [COLOR"];
2'. Reduktion des in Schritt 1' erhaltenen Produkts;
3'. Diazotierung des in Schritt 2' erhaltenen Produkts, um ein Diazoniumsalz zu erhalten;
4'. Gegebenenfalls: Nukleophile Substitution an der funktionellen Diazoniumgruppe des in Schritt 3' erhaltenen Produkts.

8. Verfahren gemäß einem der Ansprüche 5 bis 7, **dadurch gekennzeichnet, dass** es eine nukleophile
Substitutionsreaktion entweder zwischen:
2'-A'-L und [COLOR']-Nu
oder zwischen:
[COLOR"]-L und Z'-A'-Nu
umfasst,
- wobei Z' und A' wie in Anspruch 1 definiert sind;
- wobei L, Nu, [COLOR'] und [COLOR"] wie in Anspruch 7 definiert sind;
- wobei [COLOR']-Nu und [COLOR"]-L gemäß dem Verfahren einer der Ansprüche 6 und 7 hergestellt werden.

9. Verfahren zur Markierung eines Zielmoleküls mit einem Marker gemäß einem der Ansprüche 1 bis 4 oder einem gemäß einem der Ansprüche 5 bis 8 hergestellten Marker.

10. Produkt der Formel: 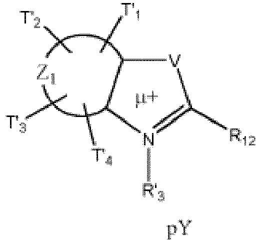 worin
Z₁, V, p, Y wie in Anspruch 1 definiert sind,
R'₃ für ein Elektronenpaar steht oder für R₃ steht, das wie in Anspruch 1 definiert ist;
µ eine ganze Zahl, gleich 0 oder 1 ist;
R₁₂ für eine lineare oder verzweigte, gesättigte oder ungesättigte C₁-C₃₀ Alkylgruppe, eine Sulfoalkylgruppe mit 1 bis 30 Kohlenstoffatomen, eine Cycloalkylgruppe mit 1 bis 30 Kohlenstoffatomen, eine Arylgruppe, oder eine Aryloxygruppe steht,
T'₁ bis T'₄ gemäß Anspruch 1 definiert sind,
unter der Voraussetzung, dass mindestens eines von T'₁ bis T'₄ für [FONC] gemäß der Definition in Anspruch 1 steht.

11. Produkt gemäß Anspruch 10, **dadurch gekennzeichnet, dass** es ausgewählt ist aus der Gruppe, bestehend aus:

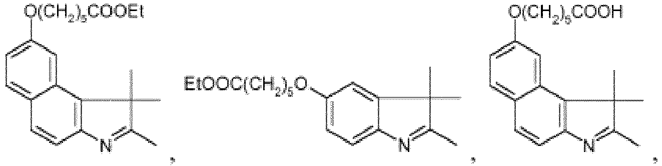

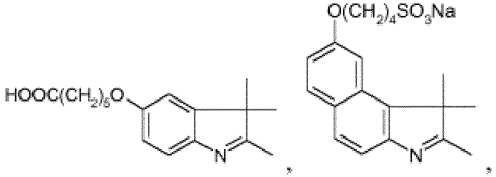

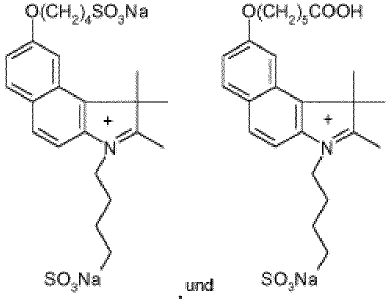

12. Verwendung eines Markers gemäß einem der Ansprüche 1 bis 4 oder eines gemäß einem der Ansprüche 5 bis 8 hergestellten Markers in einem Verfahren zur Markierung von biologischen Molekülen.

13. Verwendung eines Markers gemäß einem der Ansprüche 1 bis 4 in einer Kupplungsreaktion in der Gegenwart eines Peptidkupplungsreagenz, das aus der Gruppe, die Reagenzien des Carbodiimide-Typs wie DCC (Dicyclohexylcarbodiimid), Carbonyldiimidazol, IDDQ (1-Isobutoxycarbonyl-2-isobutoxy-1,2-dihydroquinolin); Reagenzien des Phosphonium-Typs wie BOP (Benzotriazol-1-yloxytris(dimethylamino)-phosphonium hexafluorophosphat); Reagenzien des Uronium-Typs wie HBTu (o-Benzotriazolyl-tetramethyluronium hexafluorophosphat) und TBTu (o-Benzotriazolyl-tetramethyluronium tetrafluoroborat); Woodward-Reagenzien wie N-Ethyl-5-phenylisoxazolium-3'-sulfonat; und Curtius-Reagenzien (Hydrazin und Nitrit) umfasst ausgewählt ist.

14. Verwendung gemäß Anspruch 12 oder 13, **dadurch gekennzeichnet, dass** die Kupplungsreaktion in der Gegenwart von Dimethylformamid abläuft.

15. Verfahren zum Nachweis eines biologischen oder nichtbiologischen Moleküls, umfassend die Kupplung eines Markers gemäß einem der Ansprüche 1 bis 4 an das Molekül, und den Nachweis des an den Marker gekoppelten Moleküls mittels Absorptionsspektrometrie, Fluoreszenzspektrometrie, Infrarotspektrometrie, Elektrophorese oder einem medizinischem bildgebenden Verfahren im nahen Infrarot oder Infrarot.

## Claims

1. Label consisting of a dye covalently bonded by one or more carbons of its chemical structure to:
- one or more [FUNC] groups, and
- optionally one or more [SOL] groups,
said label having the following general formula (7): 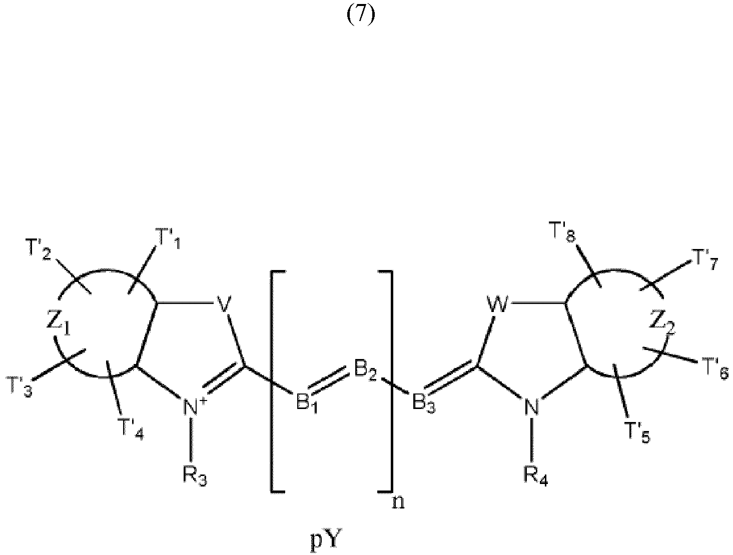
in which
- Z₁ and Z₂ each represent, independently of one another, a benzo or naphtho moiety;
- V and W, independently of one another, are selected from CR₇R₈ where R₇ and R₈, independently of one another, are each selected from hydrogen and a (CH₂)ₘR₁₀ group, where m is an integer from 1 to 18 and R₁₀ is selected from hydrogen, amine, quaternary ammonium, aldehyde, halogen, cyano, aryl, heteroaryl, hydroxyl, amide, sulphonic acid and its salts, -COOH;
- n is an integer from 1 to 10;
- R₃ and R₄ each represent, independently of one another, a hydrogen atom, a linear or branched C₁-C₃₀ alkyl group, cycloalkyl having 1 to 30 carbon atoms, aryl, aryloxy, nitroalkyl having 1 to 30 carbon atoms, alkylamine, quaternary alkylammonium having 1 to 30 carbon atoms, alkylphosphate having 1 to 30 carbon atoms, alkylsulphonic acid having 1 to 30 carbon atoms and its salts;
- T'₁ to T'₈ each represent, independently of one another:
• a hydrogen atom, a halogen atom, a linear or branched C₁ - C₃₀ alkyl group, sulphoalkyl having 1 to 30 carbon atoms, cycloalkyl having 1 to 30 carbon atoms, aryl, aryloxy, nitro, amine, quaternary ammonium, phosphate, sulphonic acid and its salts, OR₁₁ with R₁₁ selected from hydrogen and a C₁-C₃₀ alkyl group, COOR₁₁ or CONHR₁₁ with R₁₁ as defined above;
• [SOL]; or
• [FUNC],
provided at least one of T'₁ to T'₈ represents [FUNC], B₁, B₂, B₃ each represent, independently of one another, a (=CH-) methine group;
- Y represents a counter-ion selected from the ions halide, p-toluene sulphonate, methane sulphonate, trifluoromethane sulphonate, perchlorate, acetate, sodium, potassium, calcium, magnesium, lithium, ammonium and trialkylammonium;
- p is an integer from 0 to 8 necessary to the neutrality of the molecule;
[FUNC] is selected from -X-(CH₂)ᵣ-COOSu, -X-(CH₂)ᵣ-COOSuSO₃Na, -S-(CH₂)ᵣ-COOH, -O-(CH₂)s-COOH, -X-(CH₂)ᵣ-COO-C₆H₄-NO₂, -X-(C₆H₄)-(CH₂)ᵣ-COOSu, - X-(CH₂)ᵣ-NHCOCH₂I, -X-(CH₂)ᵣ-NCS, -X-(CH₂)ᵣ-C₆H₄CH(CH₃)-COOSu and-X-(CH₂)ᵣ-OP[N(iPr)₂][CH₂CH₂CN], X being an oxygen atom or a sulphur atom, r being an integer from 1 to 18, s being an integer from 3 to 18 and Su representing the succinimidyl group;
[SOL] representing, each independently, an -X'-A'-Z' group, in which:
- X' is an oxygen atom or a sulphur atom;
- A' is an alkylene group or an alkylene-arylene group;
- Z' is selected from the group comprising the groups sulphonic acid and its salts, quaternary ammonium, carbohydrate, glycol, hydroxyl, nitro, phosphate, linear or branched alkyl having 1 to 30 carbon atoms, cycloalkyl having 3 to 14 carbon atoms, alkoxy having 1 to 30 carbon atoms, halogenoalkyl having 1 to 30 carbon atoms, hydroxyalkyl having 1 to 30 carbon atoms, alkylester having 2 to 40 carbon atoms, aryl, aryloxy, aryl-(alkyl having 1 to 30 carbon atoms) and halogenoaryl;
**characterised in that** the alkylene group is a cyclic, linear or branched hydrocarbon chain having two free bonds and comprising 1 to 30 carbon atoms, and **in that** the arylene group is an aromatic group having two free bonds and comprising one or more aromatic cycles, optionally substituted.

2. Label as claimed in claim 1, **characterised in that** [SOL] is selected from X-(CH₂)ᵣ-SO₃Na, -X-(CH₂)ᵣ-SO₃H, -X-(CH₂)ᵣ-C₆H₃(NO₂)₂ and -X-(CH₂)ᵣ-CH₃, X being as defined in claim 1 and r being an integer from 1 to 18.

3. Label as claimed in claim 1 or 2, **characterised in that** it is selected from the group comprising
1) 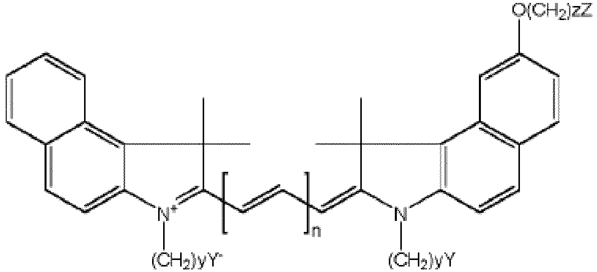
in which each of y and n, identical or different, is an integer equal to 1, 2, 3, 4, 5, 6, 7 or 8, z represents an integer equal to 3, 4, 5, 6, 7 or 8, Z represents - COOH or 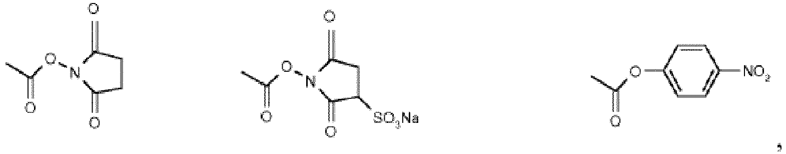
and Y represents SO₃ or SO₃Na,
2) 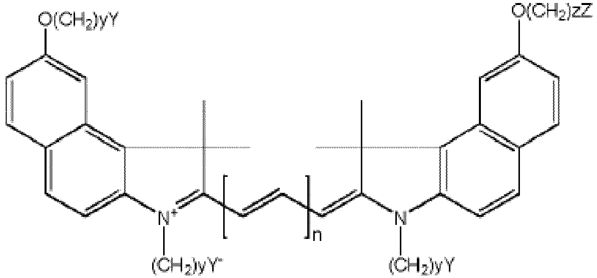
in which each of y and z, identical or different, is an integer equal to 3, 4, 5, 6, 7 or 8, n is an integer equal to 1, 2, 3, 4, 5, 6, 7 or 8,
Z represents -COOH or 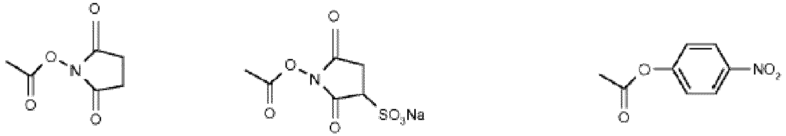
and Y represents SO₃ or SO₃Na.

4. Label as claimed in claim 3, **characterised in that** it is selected from the group comprising
1) 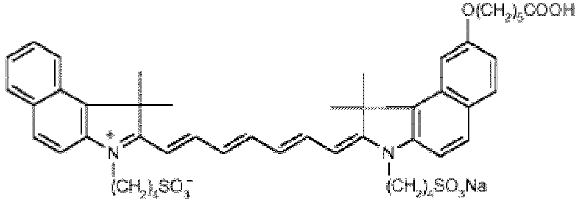 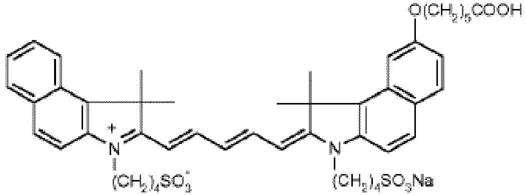 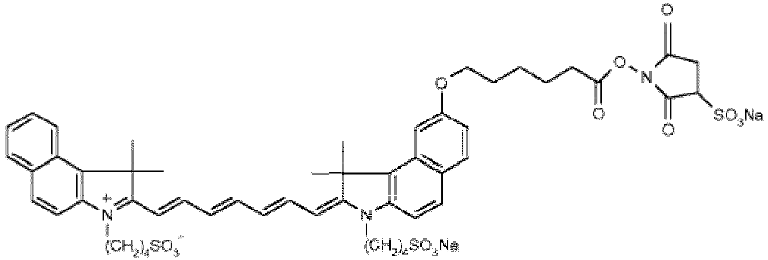 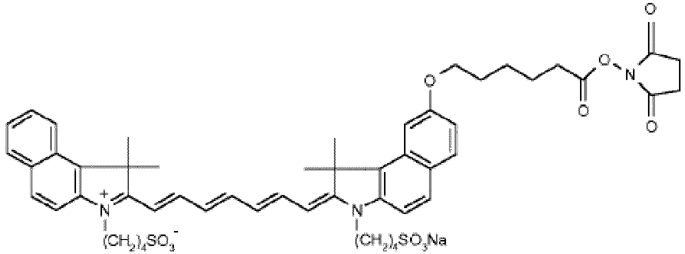 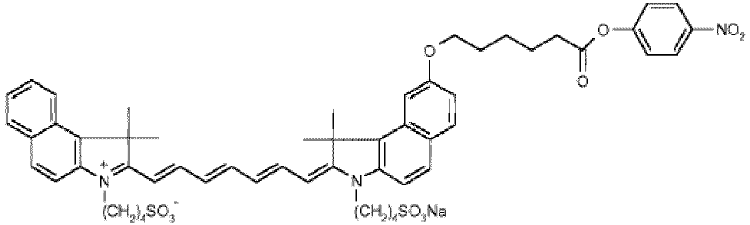 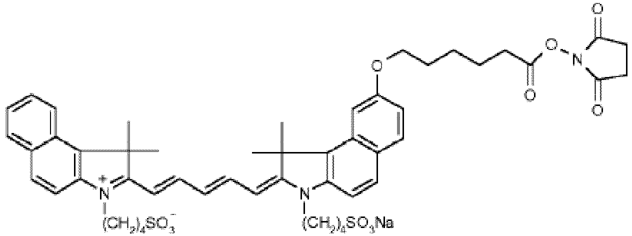
2)

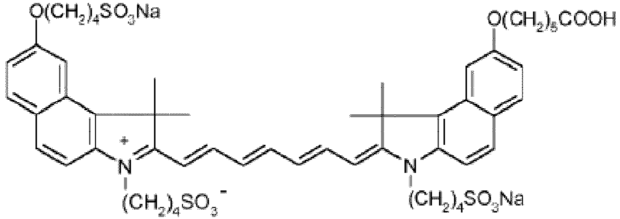

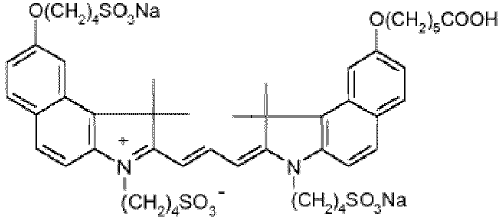

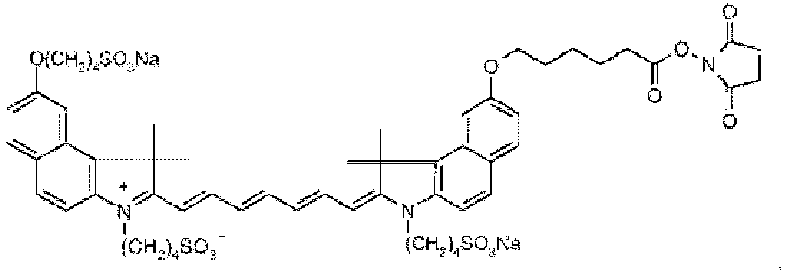

5. Method for preparing labels as claimed in any one of claims 1 to 4, **characterised in that** it comprises a nucleophile substitution reaction between:
either:
Z-A-L and [DYE']-Nu
or:
[DYE"]-L and Z-A-Nu
- Z and A being as defined in claim 1;
- L representing a leaving group selected from the group comprising a halogen, a methane sulphonate group, para-toluene sulphonate and a diazonium group;
- Nu representing a nucleophilic group selected from the group comprising -OH, -SH and -NR₁R₂, R₁ and R₂ each being, independently of one another, a hydrogen atom or a linear or branched C₁-C₃₀ alkyl group;
[DYE'] and [DYE"] representing the dye as defined in claim 1; 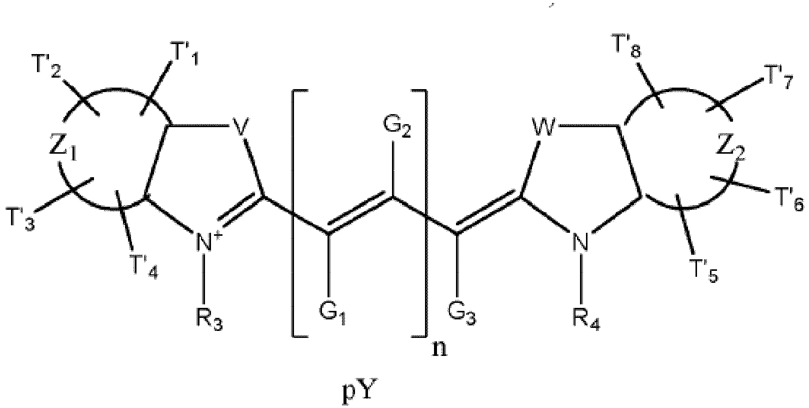
- in which none of T'1 to T'8 represent [SOL] or represent [FUNC];
the nucleophile substitution reaction being a Williamson reaction in the presence of a base.

6. Method as claimed in claim 5, **characterised in that** [DYE']-Nu is obtained by the following successive reactions:
1. nitration of [DYE'];
2. reduction of the product obtained in step 1;
3. optionally: either alkylation of the product obtained in step 2 or diazotation of the product obtained in step 2 to obtain a diazonium salt followed by nucleophilic substitution on this diazonium function.

7. Method as claimed in claim 5 or 6, **characterised in that** [DYE"]-L is obtained by the following successive reactions:
1'. nitration of [DYE"];
2'. reduction of the product obtained in step 1';
3'. diazotation of the product obtained in step 2' to obtain a diazonium salt;
4'. optionally: nucleophilic substitution on the diazonium function of the product obtained in step 3'.

8. Method as claimed in any one of claims 5 to 7, **characterised in that** it comprises a nucleophile substitution reaction between:
either:
Z'-A'-L and [DYE']-Nu
or:
[DYE"]-L and Z'-A'-Nu
- Z' and A' being as defined in claim 1;
- L, Nu, [DYE'] and [DYE"] being as defined in claim 7;
- [DYE']-Nu and [DYE"]-L being prepared by the method as claimed in any one of claims 6 and 7.

9. Method of labelling a target molecule with a label as claimed in any one of claims 1 to 4 or prepared as claimed in any one of claims 5 to 8.

10. Product having formula: 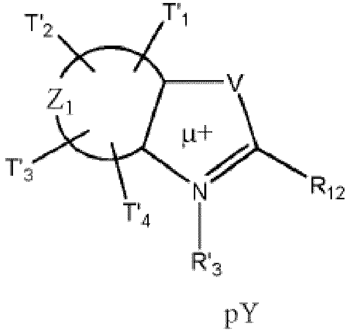 in which
Z₁, V, p, Y are as defined in claim 1,
R'₃ represents an electronic doublet or represents R₃ as defined in claim 1;
µ is an integer equal to 0 or 1;
R₁₂ represents a linear or branched, saturated or unsaturated C₁-C₃₀ alkyl group, sulphoalkyl having 1 to 30 carbon atoms, cycloalkyl having 1 to 30 carbon atoms, aryl, aryloxy,
T'₁ to T'₄ being as defined in claim 1,
provided that at least one of T'₁ to T'₄ represents [FUNC] as defined in claim 1.

11. Product as claimed in claim 10, **characterised in that** it is selected from the group comprising:

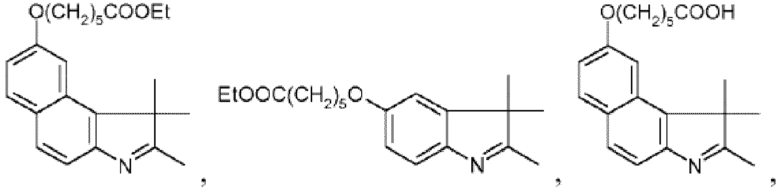

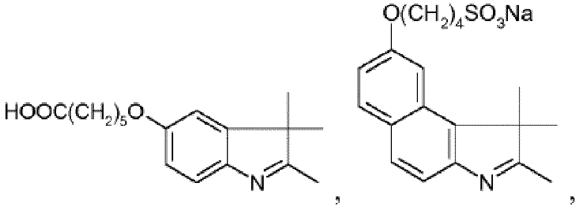

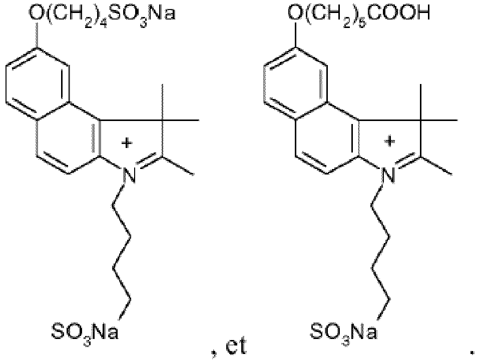

12. Use of a label as claimed in any one of claims 1 to 4 or prepared as claimed in one of claims 5 to 8 in a method for labelling biological molecules.

13. Use of a label as claimed in any one of claims 1 to 4 in a coupling reaction in the presence of a peptide coupling agent selected from the group comprising reagents of the carbodiimide type such as DCC (dicyclohexylcarbodiimide), carbonyldiimidazole, IDDQ (1-isobutyloxycarbonyl-2-isobutyloxy-1,2-dihydroquinoline); reagents of the phosphonium type such as BOP (benzotriazol-1-yloxytris-(dimethylamino)phosphonium hexafluorophosphate); reagents of the uronium type such as HBTu (o-benzotriazolyl-tetramethyluronium hexafluorophosphate) and TBTu (o-benzotriazolyl-tetramethyluronium tetrafluoroborate); Woodward reagents such as N-ethyl-5-phenylisoxazolium-3'- sulphonate; and Curtius reagents (hydrazine and nitrite).

14. Use as claimed in claim 12 or 13, **characterised in that** the coupling reaction takes place in the presence of dimethylformamide.

15. Method for detecting a biological or non-biological molecule comprising coupling a label as claimed in one of claims 1 to 4 with said molecule and detecting said molecule coupled with the marker by absorption spectrometry, fluorescence spectrometry, infrared spectrometry, electrophoresis, medical imaging in the near infrared or infrared.
